(19) Europäisches Patentamt
European Patent Office
Office européen des brevets

(11) Veröffentlichungsnummer : **0 581 725 A1**

(12) # EUROPÄISCHE PATENTANMELDUNG

(21) Anmeldenummer : **93810461.9**

(22) Anmeldetag : **29.06.93**

(51) Int. Cl.[5] : **C07C 311/51,** C07C 309/65, C07D 317/46, C07C 251/86, A01N 41/04, A01N 41/06, A01N 43/30, A01N 35/10

(30) Priorität : **07.07.92 CH 2147/92**

(43) Veröffentlichungstag der Anmeldung : **02.02.94 Patentblatt 94/05**

(84) Benannte Vertragsstaaten : **AT BE CH DE DK ES FR GB GR IE IT LI LU MC NL PT SE**

(71) Anmelder : **CIBA-GEIGY AG
Klybeckstrasse 141
CH-4002 Basel (CH)**

(72) Erfinder : **Hall, Roger Graham, Dr.
Hauptstrasse 5
CH-4147 Aesch (CH)**
Erfinder : **Pascual, Alfons, Dr.
Gundeldingerstrasse 433
CH-4053 Basel (CH)**
Erfinder : **Kristiansen, Odd, Dr.
Delligrabenstrasse 7
CH-4313 Möhlin (CH)**

(54) **Benzophenonhydrazone.**

(57) Verbindungen der Formel

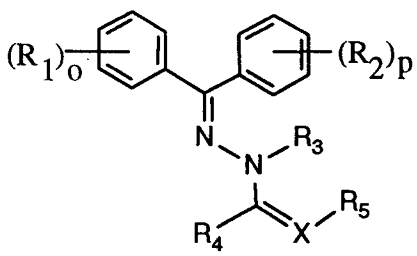

(I),

worin o und p unabhängig voneinander 0, 1, 2, 3, 4 oder 5, wobei, wenn o grösser als 1 ist, die Reste $R_1$ gleich oder verschieden sind und wobei, wenn p grösser als 1 ist, die Reste $R_2$ gleich oder verschieden sind ;
$R_1$ und $R_2$ unabhängig voneinander $C_1$-$C_4$-Alkyl, Halogen-$C_1$-$C_4$-alkyl, Halogen, $-NO_2$, -OH, $C_1$-$C_4$-Alkoxy, Halogen-$C_1$-$C_4$-alkoxy, $C_1$-$C_4$-Alkylthio, Halogen-$C_1$-$C_4$-alkylthio, $-O-S(=O)-R_6$ ,$-O-S(=O)_2-R_6$, Phenoxy oder $-N(R_{11})SO_2R_{12}$ und/oder zwei Substituenten $R_1$ und/oder zwei Substituenten $R_2$ unabhängig voneinander gemeinsam -Y-Z-Y- ;
$R_3$ Wasserstoff, $C_1$-$C_4$-Alkyl oder Halogen-$C_1$-$C_4$-alkyl ;
$R_4$ Wasserstoff, $C_1$-$C_4$-Alkyl, Halogen-$C_1$-$C_4$-alkyl, unsubstituiertes oder ein- oder zweifach substituiertes Phenyl oder Naphthyl ;
$R_5$ $-S-R_7$, $-S(=O)-R_7$, $-S(=O)_2-R_7$, $-NO_2$, -CN, $-C(=O)-R_6$ oder $-C(=O)-OR_6$ ;
$R_6$ $C_1$-$C_8$-Alkyl, oder Halogen-$C_1$-$C_8$-alkyl oder Phenyl ;
X N oder $C(R_9)$ ;
Y unabhängig voneinander O oder S und
Z Methylen, Eth-1,2-ylen, Halogenmethylen oder Halogeneth-1,2-ylen bedeuten ;
sowie ihre Tautomeren und Salze können als Schädlingsbekämpfungsmittel verwendet werden und sind in an sich bekannter Weise herstellbar.

EP 0 581 725 A1

Die Erfindung betrifft Verbindungen der Formel

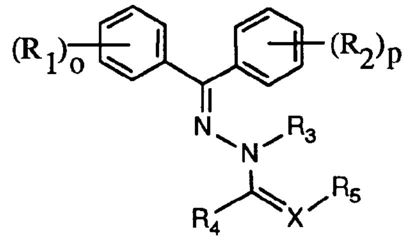

(I),

worin

o und p unabhängig voneinander 0, 1, 2, 3, 4 oder 5, wobei, wenn o grösser als 1 ist, die Reste $R_1$ gleich oder verschieden sind und wobei, wenn p grösser als 1 ist, die Reste $R_2$ gleich oder verschieden sind;

$R_1$ und $R_2$ unabhängig voneinander $C_1$-$C_4$-Alkyl, Halogen-$C_1$-$C_4$-alkyl, Halogen, -$NO_2$, -OH, $C_1$-$C_4$-Alkoxy, Halogen-$C_1$-$C_4$-alkoxy, $C_1$-$C_4$-Alkylthio, Halogen-$C_1$-$C_4$-alkylthio, -O-S(=O)-$R_6$ ,-O-S(=O)$_2$-$R_6$, Phenoxy oder -N($R_{11}$)$SO_2R_{12}$ und/oder zwei an benachbarte C-Atome des Phenylrings gebundene Substituenten $R_1$ und/oder zwei an benachbarte C-Atome des Phenylrings gebundene Substituenten $R_2$ unabhängig voneinander gemeinsam -Y-Z-Y-;

$R_3$ Wasserstoff, $C_1$-$C_4$-Alkyl oder Halogen-$C_1$-$C_4$-alkyl;

$R_4$ Wasserstoff, $C_1$-$C_4$-Alkyl, Halogen-$C_1$-$C_4$-alkyl, unsubstituiertes Phenyl oder Naphthyl oder ein- oder zweifach substituiertes Phenyl oder Naphthyl, wobei die Substituenten ausgewählt sind aus der Gruppe, bestehend aus Halogen, $C_1$-$C_4$-Alkyl, Halogen-$C_1$-$C_4$-alkyl, $C_1$-$C_4$-Alkoxy, Halogen-$C_1$-$C_4$-alkoxy, $C_1$-$C_4$-Alkylthio, Halogen-$C_1$-$C_4$-alkylthio, -$NO_2$ und -CN;

$R_5$ -S-$R_7$, -S(=O)-$R_7$, -S(=O)$_2$-$R_7$, -$NO_2$, -CN, -C(=O)-$R_5$ oder -C(=O)-O$R_8$;

$R_5$ $C_1$-$C_8$-Alkyl, oder Halogen-$C_1$-$C_8$-alkyl oder Phenyl;

$R_7$ $C_1$-$C_8$-Alkyl, $C_3$-$C_6$-Cycloalkyl, Halogen-$C_1$-$C_8$-alkyl, unsubstituiertes oder ein- oder zweifach substituiertes Phenyl, wobei die Substituenten ausgewählt sind aus der Gruppe, bestehend aus Halogen, $C_1$-$C_4$-Alkyl, Halogen-$C_1$-$C_4$-alkyl, $C_1$-$C_4$-Alkoxy, Halogen-$C_1$-$C_4$-alkoxy, $C_1$-$C_4$-Alkylthio, Halogen-$C_1$-$C_4$-alkylthio, -$NO_2$ und -CN; Benzyl oder unsubstituiertes oder ein- oder zweifach substituiertes Amino, wobei die Substituenten ausgewählt sind aus der Gruppe, bestehend aus $C_1$-$C_4$-Alkyl, Halogen-$C_1$-$C_4$-alkyl oder Phenyl;

$R_5$ $C_1$-$C_8$-Alkyl, Halogen-$C_1$-$C_8$-alkyl oder unsubstituiertes oder ein- oder zweifach substituiertes Phenyl, wobei die Substituenten ausgewählt sind aus der Gruppe, bestehend aus Halogen, $C_1$-$C_4$-Alkyl, Halogen-$C_1$-$C_4$-alkyl, $C_1$-$C_4$-Alkoxy, Halogen-$C_1$-$C_4$-alkoxy, $C_1$-$C_4$-Alkylthio, Halogen-$C_1$-$C_4$-alkylthio, -$NO_2$ und -CN;

X N oder C($R_9$);

Y unabhängig voneinander O oder S;

Z Methylen, Eth-1,2-ylen, Halogenmethylen oder Halogeneth-1,2-ylen;

$R_9$ Wasserstoff, $C_1$-$C_4$-Alkyl, Halogen-$C_1$-$C_4$-alkyl, -CN, -C(=O)-$R_{10}$ oder -C(=O)-O$R_{10}$;

$R_{10}$ Wasserstoff, $C_1$-$C_8$-Alkyl oder Halogen-$C_1$-$C_8$-alkyl;

$R_{11}$ Wasserstoff, $C_1$-$C_4$-Alkyl oder Halogen-$C_1$-$C_4$-alkyl; und

$R_{12}$ $C_1$-$C_4$-Alkyl, Halogen-$C_1$-$C_4$-alkyl, unsubstituiertes Phenyl oder ein- oder zweifach substituiertes Phenyl bedeuten, wobei die Substituenten ausgewählt sind aus der Gruppe bestehend aus Halogen, $C_1$-$C_4$-Alkyl, Halogen-$C_1$-$C_4$-alkyl, $C_1$-$C_4$-Alkoxy, Halogen-$C_1$-$C_4$-alkoxy, $C_1$-$C_4$-Alkylthio, Halogen-$C_1$-$C_4$-alkylthio, -$NO_2$ und -CN;

und gegebenenfalls Tautomere davon, sowie deren Salze und die Salze der Tautomeren; mit der Massgabe (A), dass in Verbindungen der Formel I in freier Form, worin o und p jeweils für 0 stehen, $R_4$ Wasserstoff ist und X für N steht, $R_3$ von Wasserstoff verschieden ist, wenn $R_5$ Methansulfonyl, unsubstituiertes Phenylsulfonyl oder 4-Methylphenylsulfonyl darstellt und

mit der weiteren Massgabe (B), dass in Verbindungen der Formel I in freier Form, worin o und p jeweils für 1 stehen, $R_1$ Methansulfonyloxy ist, $R_2$ Chlor ist, $R_4$ Methyl ist, X für C($R_9$) steht und $R_9$ Wasserstoff ist, $R_3$ von Wasserstoff verschieden ist, wenn $R_5$ Ethoxycarbonyl, Methoxycarbonyl oder Cyano darstellt;

ein Verfahren zur Herstellung und die Verwendung dieser Verbindungen und Tautomeren, Schädlingsbekämpfungsmittel, deren Wirkstoff aus diesen Verbindungen und Tautomeren, oder deren agrochemisch verwendbare Salze, ausgewählt ist, und ein Verfahren zur Herstellung und die Verwendung dieser Mittel.

Die Verbindungen der Formel I können teilweise als Tautomere vorliegen. Bedeutet z. B. $R_3$ Wasserstoff und steht X für N, können entsprechende Verbindungen der Formel I, also solche mit einer -N(H)-C($R_4$)=N-$R_5$-Teilstruktur, im Gleichgewicht mit den jeweiligen Tautomeren vorliegen, die eine -N=C($R_4$)-N(H)-$R_5$-Teilstruktur aufweisen. Demgemäss sind unter den Verbindungen der Formel I nachstehend gegebenenfalls auch entspre-

chende Tautomere zu verstehen, auch wenn letztere nicht in jedem Fall speziell erwähnt werden.

Die Verbindungen der Formel I und gegebenenfalls ihre Tautomeren können als Salze vorliegen. Verbindungen der Formel I, welche mindestens ein basisches Zentrum aufweisen, können z. B. Säureadditionssalze bilden. Diese werden beispielsweise mit starken anorganischen Säuren, wie Mineralsäuren, z. B. Schwefelsäure, einer Phosphorsäure oder einer Halogenwasserstoffsäure, mit starken organischen Carbonsäuren, wie gegebenenfalls, z. B. durch Halogen, substituierten $C_1$-$C_4$-Alkancarbonsäuren, z. B. Essigsäure, wie gegebenenfalls ungesättigten Dicarbonsäuren, z. B. Oxal-, Malon-, Malein-, Fumar- oder Phthalsäure, wie Hydroxycarbonsäuren, z. B. Ascorbin-, Milch-, Äpfel-, Wein- oder Zitronensäure, oder wie Benzoesäure, oder mit organischen Sulfonsäuren, wie gegebenenfalls, z. B. durch Halogen, substituierten $C_1$-$C_4$-Alkan- oder Arylsulfonsäuren, z. B. Methan- oder p-Toluolsulfonsäure, gebildet. Ferner können Verbindungen der Formel I mit mindestens einer aciden Gruppe Salze mit Basen bilden. Geeignete Salze mit Basen sind beispielsweise Metallsalze, wie Alkali- oder Erdalkalimetallsalze, z. B. Natrium-, Kalium- oder Magnesiumsalze, oder Salze mit Ammoniak oder einem organischen Amin, wie Morpholin, Piperidin, Pyrrolidin, einem Mono-, Di- oder Triniederalkylamin, z. B. Ethyl-, Diethyl-, Triethyl- oder Dimethyl-propyl-amin, oder einem Mono-, Di- oder Trihydroxyniederalkylamin, z. B. Mono-, Di- oder Triethanolamin. Weiterhin können gegebenenfalls entsprechende innere Salze gebildet werden. Bevorzugt sind im Rahmen der Erfindung agrochemisch vorteilhafte Salze; umfasst sind aber auch für agrochemische Verwendungen mit Nachteilen behaftete Salze, welche jedoch beispielsweise für die Isolierung bzw. Reinigung von freien Verbindungen der Formel I oder deren agrochemisch verwendbaren Salzen eingesetzt werden können. Der Begriff "Verbindung der Formel I" umfasst also vor- und nachstehend auch die Salze dieser Verbindungen, die Tautomeren dieser Verbindungen sowie die Salze der Tautomeren.

Halogen - als Substituent per se sowie als Strukturelement von anderen Gruppen und Verbindungen, wie von Halogenalkyl, Halogenalkoxy, Halogenalkylthio, Halogenmethylen und Halogeneth-1,2-ylen, - ist Fluor, Chlor, Brom oder Iod, insbesondere Fluor, Chlor oder Brom, vor allem Fluor oder Chlor.

Kohlenstoffhaltige Gruppen und Verbindungen enthalten, sofern nicht abweichend definiert, jeweils 1 bis und mit 8, vorzugsweise 1 bis und mit 4, insbesondere 1 oder 2, Kohlenstoffatome.

$C_3$-$C_6$-Cycloalkyl ist Cyclopropyl, Cyclobutyl, Cyclopentyl oder Cyclohexyl.

Alkyl - als Gruppe per se sowie als Strukturelement von anderen Gruppen und Verbindungen, wie von Halogenalkyl, Alkoxy, Halogenalkoxy, Alkylthio und Halogenalkylthio, - ist, jeweils unter gebührender Berücksichtigung der von Fall zu Fall umfassten Anzahl der in der entsprechenden Gruppe oder Verbindung enthaltenen Kohlenstoffatome, entweder geradkettig, d. h. Methyl, Ethyl, Propyl, Butyl, Pentyl, Hexyl, Heptyl oder Octyl, oder verzweigt, z. B. Isopropyl, Isobutyl, sek.-Butyl, tert.-Butyl, Isopentyl, Neopentyl oder Isooctyl.

Halogen-substituierte kohlenstoffhaltige Gruppen und Verbindungen, wie Halogenalkyl, Halogenalkoxy, Halogenalkylthio, Halogenmethylen und Halogeneth-1,2-ylen, können teilweise halogeniert oder perhalogeniert sein, wobei im Falle von Mehrfach-Halogenierung die Halogensubstituenten gleich oder verschieden sein können. Beispiele für Halogenalkyl - als Gruppe per se sowie als Strukturelement von anderen Gruppen und Verbindungen, wie von Halogenalkylthio und Halogenalkoxy, - sind das ein- bis dreifach durch Fluor, Chlor und/oder Brom substituierte Methyl, wie $CHF_2$ oder $CF_3$; das ein- bis fünffach durch Fluor, Chlor und/oder Brom substituierte Ethyl, wie $CH_2CF_3$, $CF_2CF_3$, $CF_2CCl_3$, $CF_2CHCl_2$, $CF_2CHF_2$, $CF_2CFCl_2$, $CF_2CHBr_2$, $CF_2CHClF$, $CF_2CHBrF$ oder $CClFCHClF$; das ein- bis siebenfach durch Fluor, Chlor und/oder Brom substituierte Propyl oder Isopropyl, wie $CH_2CHBrCH_2Br$, $CF_2CHFCF_3$, $CH_2CF_2CF_3$ oder $CH(CF_3)_2$; und das ein- bis neunfach durch Fluor, Chlor und/oder Brom substituierte Butyl oder eines seiner Isomeren, wie $CF(CF_3)CHFCF_3$ oder $CH_2(CF_2)_2CF_3$. Beispiele für Halogenmethylen sind Fluormethylen, Difluormethylen und Dichlormethylen. Beispiele für Halogeneth-1,2-ylen sind 1,2-Difluoreth-1,2-ylen, Tetrafluoreth-1,2-ylen und Tetrachloreth-1,2-ylen.

Bevorzugte Ausführungsformen im Rahmen der Erfindung, jeweils unter Berücksichtigung der vorstehend erwähnten Massgaben (A) und (B), sind:

(1) Eine Verbindung der Formel I, worin

o und p unabhängig voneinander 0, 1, 2, 3, 4 oder 5, wobei, wenn o grösser als 1 ist, die Reste $R_1$ gleich oder verschieden sind und wobei, wenn p grösser als 1 ist, die Reste $R_2$ gleich oder verschieden sind;

$R_1$ und $R_2$ unabhängig voneinander $C_1$-$C_4$-Alkyl, Halogen-$C_1$-$C_4$-alkyl, Halogen, -OH, $C_1$-$C_4$-Alkoxy, Halogen-$C_1$-$C_4$-alkoxy, $C_1$-$C_4$-Alkylthio, Halogen-$C_1$-$C_4$-alkylthio, $-O-S(=O)-R_6$ oder $-O-S(=O)_2-R_6$ und/oder zwei an benachbarte C-Atome des Phenylrings gebundene Substituenten $R_1$ und/oder zwei an benachbarte C-Atome des Phenylrings gebundene Substituenten $R_2$ unabhängig voneinander gemeinsam -Y-Z-Y-;

$R_3$ Wasserstoff, $C_1$-$C_4$-Alkyl oder Halogen-$C_1$-$C_4$-alkyl;

$R_4$ Wasserstoff, $C_1$-$C_4$-Alkyl, Halogen-$C_1$-$C_4$-alkyl, unsubstituiertes Phenyl oder Naphthyl oder ein- oder zweifach substituiertes Phenyl oder Naphthyl, wobei die Substituenten ausgewählt sind aus der Gruppe,

bestehend aus Halogen, $C_1$-$C_4$-Alkyl, Halogen-$C_1$-$C_4$-alkyl, $C_1$-$C_4$-Alkoxy, Halogen-$C_1$-$C_4$-alkoxy, $C_1$-$C_4$-Alkylthio, Halogen-$C_1$-$C_4$-alkylthio, -$NO_2$ und -CN;

$R_5$ -S-$R_7$, -S(=O)-$R_7$, -$S(=O)_2$-$R_7$, -$NO_2$, -CN, -C(=O)-$R_8$ oder -C(=O)-$OR_8$;

$R_6$ $C_1$-$C_8$-Alkyl oder Halogen-$C_1$-$C_8$-alkyl;

$R_7$ $C_1$-$C_8$-Alkyl, $C_3$-$C_6$-Cycloalkyl, Halogen-$C_1$-$C_8$-alkyl oder unsubstituiertes oder ein- oder zweifach substituiertes Phenyl, wobei die Substituenten ausgewählt sind aus der Gruppe, bestehend aus Halogen, $C_1$-$C_4$-Alkyl, Halogen-$C_1$-$C_4$-alkyl, $C_1$-$C_4$-Alkoxy, Halogen-$C_1$-$C_4$-alkoxy, $C_1$-$C_4$-Alkylthio, Halogen-$C_1$-$C_4$-alkylthio, -$NO_2$ und -CN;

$R_8$ $C_1$-$C_8$-Alkyl, Halogen-$C_1$-$C_8$-alkyl oder unsubstituiertes oder ein- oder zweifach substituiertes Phenyl, wobei die Substituenten ausgewählt sind aus der Gruppe, bestehend aus Halogen, $C_1$-$C_4$-Alkyl, Halogen-$C_1$-$C_4$-alkyl, $C_1$-$C_4$-Alkoxy, Halogen-$C_1$-$C_4$-alkoxy, $C_1$-$C_4$-Alkylthio, Halogen-$C_1$-$C_4$-alkylthio, -$NO_2$ und -CN;

X N oder C($R_9$);

Y unabhängig voneinander O oder S;

Z Methylen, Eth-1,2-ylen, Halogenmethylen oder Halogeneth-1,2-ylen;

$R_9$ Wasserstoff, $C_1$-$C_4$-Alkyl, Halogen-$C_1$-$C_4$-alkyl, -CN, -C(=O)-$R_{10}$ oder -C(=O)-$OR_{10}$; und

$R_{10}$ $C_1$-$C_8$-Alkyl oder Halogen-$C_1$-$C_8$-alkyl bedeuten;

und gegebenenfalls Tautomere davon,

(2) Eine Verbindung der Formel I, worin o 1 oder 2 ist, wobei, wenn o 2 ist, die Reste $R_1$ gleich oder verschieden sind, und

$R_1$ Halogen, -$NO_2$, -OH, -O-$S(=O)_2$-$C_1$-$C_4$-Alkyl, -O-$S(=O)_2$-Halogen-$C_1$-$C_4$-alkyl, Phenoxy, $NO_2$ oder -N($R_{11}$)$SO_2R_{12}$, oder zwei an benachbarte C-Atome des Phenylrings gebundene Substituenten $R_1$ gemeinsam -O-Methylen-O- oder -O-Halogenmethylen-O-, $R_{11}$ Wasserstoff oder $C_1$-$C_4$-Alkyl, und $R_{12}$ Halogen-$C_1$-$C_4$-alkyl;

besonders worin $R_1$ Halogen, -OH, -O-$S(=O)_2$-$C_1$-$C_4$-Alkyl, -O-$S(=O)_2$-Halogen-$C_1$-$C_4$-alkyl, Phenoxy, -N($R_{11}$)$SO_2$-halogen-$C_1$-$C_2$-alkyl oder zwei an benachbarte C-Atome des Phenylrings gebundene Substituenten $R_1$ gemeinsam -O-Methylen-O- oder -O-Halogenmethylen-O- und $R_{11}$ Wasserstoff oder $C_1$-$C_2$-alkyl;

ganz besonders o 1 oder 2, wobei, wenn o 2 ist, die Reste $R_1$ gleich sind, und $R_1$ Halogen, -OH, -O-$S(=O)_2$-$C_1$-$C_2$-Alkyl, -O-$S(=O)_2$-Halogen-$C_1$-$C_2$-alkyl, -N($C_2H_5$)$SO_2CF_3$ oder zwei an benachbarte C-Atome des Phenylrings gebundene Substituenten $R_1$ gemeinsam -O-Halogenmethylen-O-;

insbesondere $(R_1)_o$ 4-Trifluormethansulfonyloxy, 4-Methansulfonyloxy oder in 3- und 4-Position gebundenes -$OCF_2O$-;

besonders bevorzugt $(R_1)_o$ 4-Trifluormethansulfonyloxy bedeuten, oder gegebenenfalls ein Tautomeres davon;

(3) Eine Verbindung der Formel I, worin p 0, 1 oder 2, wobei, wenn p 2 ist, die Reste $R_2$ gleich oder verschieden sind, und

$R_2$ Halogen, -OH, -O-$S(=O)_2$-$C_1$-$C_4$-Alkyl oder -O-$S(=O)_2$-Halogen-$C_1$-$C_4$-alkyl oder zwei an benachbarte C-Atome des Phenylrings gebundene Substituenten $R_2$ gemeinsam -O-Methylen-O- oder -O-Halogenmethylen-O-;

besonders p 0, 1 oder 2, wobei, wenn p 2 ist, die Reste $R_2$ gleich sind, und $R_2$ Halogen, -OH, -O-$S(=O)_2$-$C_1$-$C_2$-Alkyl oder -O-$S(=O)_2$-Halogen-$C_1$-$C_2$-alkyl oder zwei an benachbarte C-Atome des Phenylrings gebundene Substituenten $R_2$ gemeinsam -O-Halogenmethylen-O-;

insbesondere $(R_2)_p$ Fluor, Chlor, Brom, $C_1$-$C_4$-Alkyl;

ganz besonders $(R_2)_p$ 2-Clor, 4-Chlor, 2,4-Dichlor, 3,4-Dichlor oder 4-Fluor;

ganz besonders bevorzugt 4-Chlor bedeuten, oder gegebenenfalls ein Tautomeres davon;

(4) Eine Verbindung der Formel I, worin

$R_3$ Wasserstoff oder $C_1$-$C_4$-Alkyl;

besonders Wasserstoff oder $C_1$-$C_2$-Alkyl;

insbesondere Wasserstoff oder Methyl;

ganz besonders Wasserstoff, bedeutet, oder gegebenenfalls ein Tautomeres davon;

(5) Eine Verbindung der Formel I, worin

$R_4$ Wasserstoff, $C_1$-$C_4$-Alkyl, Halogen-$C_1$-$C_4$-alkyl oder Phenyl;

besonders Wasserstoff, $C_1$-$C_2$-Alkyl, Halogen-$C_1$-$C_2$-alkyl oder Phenyl;

insbesondere Wasserstoff oder Methyl;

ganz besonders Wasserstoff bedeutet, oder gegebenenfalls ein Tautomeres davon;

(6) Eine Verbindung der Formel I, worin

$R_5$ -$S(=O)_2$-$R_7$, -CN, -C(=O)-$R_8$ oder -C(=O)-$OR_8$,

$R_7$ $C_1$-$C_6$-Alkyl, $C_3$-$C_6$-Cycloalkyl, Halogen-$C_1$-$C_4$-alkyl, Benzyl, $C_1$-$C_4$-Dialkylamino oder unsubstituiertes oder einfach durch Halogen, $C_1$-$C_4$-Alkyl oder $C_1$-$C_4$-Alkoxy substituiertes Phenyl,
$R_8$ $C_1$-$C_4$-Alkyl, Halogen-$C_1$-$C_4$-alkyl oder Phenyl,
X N oder C($R_9$),
$R_9$ Wasserstoff, -CN oder -C(=O)-O$R_{10}$ und $R_{10}$ $C_1$-$C_4$-Alkyl; besonders
$R_5$ -S(=O)$_2$-$R_7$, -CN, -C(=O)-$R_8$ oder -C(=O)-O$R_8$,
$R_7$ $C_1$-$C_4$-Alkyl, Halogen-$C_1$-$C_2$-alkyl, Benzyl oder Dimethylamino, oder unsubstituiertes oder einfach durch Halogen, $C_1$-$C_2$-Alkyl oder $C_1$-$C_2$-Alkoxy substituiertes Phenyl, $R_8$ $C_1$-$C_4$-Alkyl oder Halogen-$C_1$-$C_2$-alkyl,
X N oder C($R_9$), $R_9$ Wasserstoff, -CN oder -C(=O)-O$R_{10}$ und $R_{10}$ $C_1$-$C_4$-Alkyl; insbesondere
$R_5$ -S(=O)$_2$-$R_7$, -CN oder -C(=O)-$R_8$,
$R_7$ $C_1$-$C_4$-Alkyl, Chlormethyl, Brommethyl oder unsubstituiertes oder in 4-Position einfach durch Halogen, $C_1$-$C_2$-Alkyl oder $C_1$-$C_2$-Alkoxy substituiertes Phenyl, Benzyl oder Dimethylamino,
$R_8$ Methyl oder Trifluormethyl,
X N oder C($R_9$) und
$R_9$ Wasserstoff bedeuten, oder gegebenenfalls ein Tautomeres davon;
(7) Eine Verbindung der Formel I, worin
o 1 oder 2,
p 0, 1 oder 2, wobei, wenn o 2 ist, die Reste $R_1$ gleich oder verschieden sind und wobei, wenn p 2 ist, die Reste $R_2$ gleich oder verschieden sind;
$R_1$ und $R_2$ unabhängig voneinander Halogen, -$NO_2$, -OH, Phenoxy, -N($R_{11}$)$SO_2CF_3$ oder -O-S(=O)$_2$-$R_6$ oder zwei an benachbarte C-Atome des Phenylrings gebundene Substituenten $R_1$ oder zwei an benachbarte C-Atome des Phenylrings gebundene Substituenten $R_2$ gemeinsam -O-Z-O-;
$R_3$ Wasserstoff oder $C_1$-$C_4$-Alkyl;
$R_4$ Wasserstoff, $C_1$-$C_4$-Alkyl, Halogen-$C_1$-$C_4$-alkyl oder unsubstituiertes Phenyl;
$R_5$ -S(=O)$_2$-$R_7$, -CN, -C(=O)-$R_8$ oder -C(=O)-O$R_8$;
$R_6$ $C_1$-$C_4$-Alkyl, Halogen-$C_1$-$C_4$-alkyl oder Phenyl;
$R_7$ $C_1$-$C_6$-Alkyl, $C_3$-$C_6$-Cycloalkyl, Halogen-$C_1$-$C_4$-alkyl, unsubstituiertes oder einfach durch Halogen, $C_1$-$C_4$-Alkyl oder $C_1$-$C_4$-Alkoxy substituiertes Phenyl, Benzyl oder Dimethylamino;
$R_8$ $C_1$-$C_4$-Alkyl oder Halogen-$C_1$-$C_4$-alkyl;
X N oder C($R_9$);
Z Methylen oder Halogenmethylen;
$R_9$ Wasserstoff, -CN, -C(=O)-$R_{10}$ oder -C(=O)-O$R_{10}$;
$R_{10}$ $C_1$-$C_4$-Alkyl und
$R_{11}$ Wasserstoff, Methyl oder Ethyl bedeuten, oder gegebenenfalls ein Tautomeres davon;
(8) Eine Verbindung der Formel I, worin
o 1 oder 2,
p 0, 1 oder 2, wobei, wenn o 2 ist, die Reste $R_1$ gleich oder verschieden sind und wobei, wenn p 2 ist, die Reste $R_2$ gleich oder verschieden sind;
$R_1$ und $R_2$ unabhängig voneinander Halogen, -OH oder -O-S(=O)$_2$-$R_6$ oder zwei an benachbarte C-Atome des Phenylrings gebundene Substituenten $R_1$ oder zwei an benachbarte C-Atome des Phenylrings gebundene Substituenten $R_2$ gemeinsam -O-Z-O-;
$R_3$ Wasserstoff oder $C_1$-$C_4$-Alkyl;
$R_4$ Wasserstoff, $C_1$-$C_4$-Alkyl, Halogen-$C_1$-$C_4$-alkyl oder unsubstituiertes Phenyl;
$R_5$ -S(=O)$_2$-$R_7$, -CN, -C(=O)-$R_8$ oder -C(=O)-O$R_8$;
$R_6$ $C_1$-$C_4$-Alkyl oder Halogen-$C_1$-$C_4$-alkyl;
$R_7$ $C_1$-$C_6$-Alkyl, $C_3$-$C_6$-Cycloalkyl, Halogen-$C_1$-$C_4$-alkyl oder unsubstituiertes oder einfach durch Halogen, $C_1$-$C_4$-Alkyl oder $C_1$-$C_4$-Alkoxy substituiertes Phenyl, Benzyl oder Dimethylamino;;
$R_8$ $C_1$-$C_4$-Alkyl oder Halogen-$C_1$-$C_4$-alkyl;
X N oder C($R_9$);
Z Methylen oder Halogenmethylen;
$R_9$ Wasserstoff, -CN, -C(=O)-$R_{10}$ oder -C(=O)-O$R_{10}$; und
$R_{10}$ $C_1$-$C_4$-Alkyl bedeuten, oder gegebenenfalls ein Tautomeres davon;
(9) Eine Verbindung der Formel I, worin
o 1 oder 2,
p 0, 1 oder 2, wobei, wenn o 2 ist, die Reste $R_1$ gleich sind und wobei, wenn p 2 ist, die Reste $R_2$ gleich sind;
$R_1$ und $R_2$ unabhängig voneinander Halogen, -OH oder -O-S(=O)$_2$-$R_6$ oder zwei an benachbarte C-Atome

des Phenylrings gebundene Substituenten $R_1$ oder zwei an benachbarte C-Atome des Phenylrings gebundene Substituenten $R_2$ gemeinsam -O-Z-O-;
$R_3$ Wasserstoff oder $C_1$-$C_2$-Alkyl;
$R_4$ Wasserstoff, $C_1$-$C_2$-Alkyl, Halogen-$C_1$-$C_2$-alkyl oder unsubstituiertes Phenyl;
$R_5$ -S(=O)$_2$-$R_7$, -CN, -C(=O)-$R_8$ oder -C(=O)-O$R_8$;
$R_6$ $C_1$-$C_2$-Alkyl oder Halogen-$C_1$-$C_2$-alkyl;
$R_7$ $C_1$-$C_4$-Alkyl, Halogen-$C_1$-$C_2$-alkyl oder unsubstituiertes oder einfach durch Halogen, $C_1$-$C_2$-Alkyl oder $C_1$-$C_2$-Alkoxy substituiertes Phenyl;
$R_8$ $C_1$-$C_4$-Alkyl oder Halogen-$C_1$-$C_2$-alkyl;
X N oder C($R_9$);
Z Halogenmethylen;
$R_9$ Wasserstoff, -CN oder -C(=O)-O$R_{10}$; und
$R_{10}$ $C_1$-$C_2$-Alkyl bedeuten, oder gegebenenfalls ein Tautomeres davon;
(10) Eine Verbindung der Formel I, worin
$(R_1)_o$ für 4-Trifluormethansulfonyloxy, 4-Methansulfonyloxy oder in 3- und 4-Position gebundenes -O$CF_2$O- steht;
$R_2$ Fluor, Chlor, Brom oder $C_1$-$C_4$-Alkyl ist;
$R_3$ Wasserstoff;
$R_4$ Wasserstoff oder Methyl;
$R_5$ -S(=O)$_2$-$R_7$;
$R_7$ $C_1$-$C_4$-Alkyl, Chlormethyl, Brommethyl oder unsubstituiertes oder in 4-Position einfach durch Halogen, $C_1$-$C_2$-Alkyl oder $C_1$-$C_2$-Alkoxy substituiertes Phenyl; und X N bedeuten, oder gegebenenfalls ein Tautomeres davon;
(11) Eine Verbindung der Formel I, worin
$(R_1)_o$ 4-Trifluormethansulfonyloxy;
$(R_2)_p$ 2-Clor, 4-Chlor, 2,4-Dichlor, 3,4-Dichlor oder 4-Fluor;
$R_3$ Wasserstoff;
$R_4$ Methyl oder Trifluormethyl;
$R_5$ -CN oder -C(=O)-$R_8$;
$R_8$ Methyl oder Trifluormethyl;
X C($R_9$); und
$R_9$ Wasserstoff oder -CN bedeuten, oder gegebenenfalls ein Tautomeres davon.

Besonders bevorzugt sind im Rahmen der Erfindung die in den Beispielen H1 bis H3 genannten Verbindungen der Formel I und gegebenenfalls deren Tautomere.

Namentlich bevorzugt sind im Rahmen der Erfindung
(a) 1-(4-Chlorphenyl)-5-ethansulfonyl-2,3,5-triaza-1-(4-trifluormethansulfonyloxyphenyl)-penta-1,4-dien bzw. 1-(4-Chlorphenyl)-5-ethansulfonyl-2,3,5-triaza-1-(4-trifluormethansulfonyloxyphenyl)-penta-1,3-dien; und
(b) 1-(4-Chlorphenyl)-2,3-diaza-4-methyl-6-oxo-1-(4-trifluormethansulfonyloxyphenyl)-hepta-1,4-dien bzw. 1-(4-Chlorphenyl)-2,3-diaza-4-methyl-6-oxo-1-(4-trifluormethan-sulfonyloxyphenyl)-hepta-1,3-dien.

Als weiterer Gegenstand der Erfindung ist, unter Berücksichtigung der vorstehend erwähnten Massgaben (A) und (B), das Verfahren zur Herstellung der Verbindungen der Formel I und gegebenenfalls ihrer Tautomeren, jeweils in freier Form oder in Salzform, z.B. dadurch gekennzeichnet, dass man
a) eine Verbindung der Formel

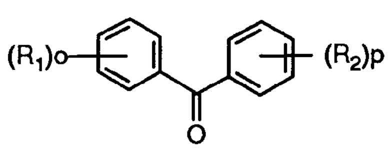

(II),

worin o, p, $R_1$ und $R_2$ die für die Formel I angegebenen Bedeutungen haben, vorzugsweise in Gegenwart einer Säure, mit einer Verbindung der Formel

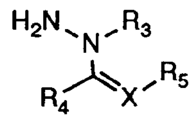

(III),

worin $R_3$, $R_4$, $R_5$ und X die für die Formel I angegebenen Bedeutungen haben, oder einem Salz und/oder gegebenenfalls einem Tautomeren davon umsetzt oder

b) eine Verbindung der Formel

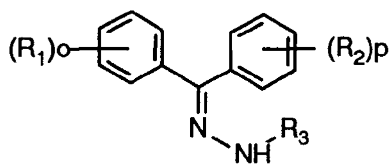

(IV),

worin o, p, $R_1$, $R_2$ und $R_3$ die für die Formel I angegebenen Bedeutungen haben, oder ein Salz davon, vorzugsweise in Gegenwart einer Säure oder einer Base, mit einer Verbindung der Formel

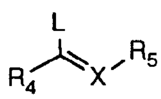

(V),

worin L Hydroxy, $C_1$-$C_8$-Alkoxy, Halogen-$C_1$-$C_8$-alkoxy, $C_1$-$C_8$-Alkanoyloxy, Mercapto, $C_1$-$C_8$-Alkylthio, Halogen-$C_1$-$C_8$-alkylthio, $C_1$-$C_8$-Alkansulfonyloxy, Halogen-$C_1$-$C_8$-alkansulfonyloxy, Benzolsulfonyloxy, Toluolsulfonyloxy oder Halogen ist und $R_4$, $R_5$ und X die für die Formel I angegebenen Bedeutungen haben, oder einem Salz und/oder gegebenenfalls einem Tautomeren davon umsetzt

und jeweils, wenn erwünscht, eine verfahrensgemäss oder auf andere Weise erhältliche Verbindung der Formel I oder ein Tautomeres davon, oder gegebenenfalls deren Salze, in eine andere Verbindung der Formel I oder ein Tautomeres davon überführt, ein verfahrensgemäss erhältliches Gemisch von Isomeren auftrennt und das gewünschte Isomere isoliert und/oder eine verfahrensgemäss erhältliche freie Verbindung der Formel I oder ein Tautomeres davon in ein Salz oder ein verfahrensgemäss erhältliches Salz einer Verbindung der Formel I oder eines Tautomeren davon in die freie Verbindung der Formel I oder ein Tautomeres davon oder in ein anderes Salz überführt.

Für vor- und nachstehend aufgeführte Ausgangsmaterialien gilt im Hinblick auf deren Tautomere bzw. Salze das vorstehend für Tautomere bzw. Salze von Verbindungen der Formel I Gesagte in analoger Weise.

Die vor- und nachstehend beschriebenen Umsetzungen werden in an sich bekannter Weise durchgeführt, z. B. in Ab- oder üblicherweise in Anwesenheit eines geeigneten Lösungs- oder Verdünnungsmittels oder eines Gemisches derselben, wobei man je nach Bedarf unter Kühlen, bei Raumtemperatur oder unter Erwärmen, z. B. in einem Temperaturbereich von etwa -80°C bis zur Siedetemperatur des Reaktionsmediums, vorzugsweise von etwa -20°C bis etwa +150°C, und, falls erforderlich, in einem geschlossenen Gefäss, unter Druck, in einer Inertgasatmosphäre und/oder unter wasserfreien Bedingungen arbeitet. Besonders vorteilhafte Reaktionsbedingungen können den Beispielen entnommen werden.

Die vor- und nachstehend aufgeführten Ausgangsmaterialien, die für die Herstellung der Verbindungen der Formel I und gegebenenfalls ihrer Tautomeren, jeweils in freier Form oder in Salzform, verwendet werden, sind bekannt oder können nach an sich bekannten Methoden, z.B. gemäss den nachstehenden Angaben, hergestellt werden.

Variante a):

Geeignete Säuren zur Erleichterung der Kondensation sind beispielsweise diejenigen, die vorstehend als für die Bildung von Säureadditionssalzen mit Verbindungen der Formel I geeignet aufgeführt sind.

Die Reaktionspartner können als solche, d. h. ohne Zusatz eines Lösungs- oder Verdünnungsmittels, z. B. in der Schmelze, miteinander umgesetzt werden. Zumeist ist jedoch der Zusatz eines inerten Lösungs- oder Verdünnungsmittels oder eines Gemisches derselben vorteilhaft. Als Beispiele für solche Lösungs- oder Verdünnungsmittel seien genannt: aromatische, aliphatische und alicyclische Kohlenwasserstoffe und Halogenkohlenwasserstoffe, wie Benzol, Toluol, Xylol, Chlorbenzol, Brombenzol, Petrolether, Hexan, Cyclohexan, Dichlormethan, Trichlormethan, Dichlorethan oder Trichlorethan; Ester, wie Essigsäureethylester; Ether, wie Diethylether, tert.-Butylmethylether, Ethylenglykolmonomethylether, Ethylenglykolmonoethylether, Ethylenglykoldimethylether, Tetrahydrofuran oder Dioxan; Ketone, wie Aceton, Methylethylketon oder Methylisobutylketon; Alkohole, wie Methanol, Ethanol, Propanol, Butanol, Ethylenglykol oder Glycerin; Amide, wie N,N-Dimethylformamid, N,N-Dimethylacetamid, N-Methylpyrrolidon oder Hexamethylphosphorsäuretriamid; Nitrile, wie Acetonitril; Sulfoxide, wie Dimethylsulfoxid; und Säuren, z. B. starke organische Carbonsäuren, wie gegebenenfalls, z. B. durch Halogen, substituierte $C_1$-$C_4$-Alkancarbonsäuren, z. B. Ameisensäure, Essigsäure oder Propionsäure.

Die Umsetzung erfolgt vorteilhaft in einem Temperaturbereich von etwa 0°C bis etwa +180°C, bevorzugt von etwa +20°C bis etwa +130°C, in vielen Fällen bei der Rückflusstemperatur des verwendeten Lösungsmittels.

Die Verbindungen der Formel II sowie die Verbindungen der Formel III und gegebenenfalls deren Tautomere sowie deren Salze, sind bekannt oder können in Analogie zu bekannten Verbindungen hergestellt werden.

Variante b:

Geeignete Säuren zur Erleichterung der HL-Abspaltung sind beispielsweise diejenigen, die vorstehend als für die Bildung von Säureadditionssalzen mit Verbindungen der Formel I geeignet aufgeführt sind.

Geeignete Basen zur Erleichterung der HL-Abspaltung sind z. B. Alkalimetall- oder Erdalkalimetall-hydroxide, -hydride, -amide, -alkanolate, -carbonate, -dialkylamide oder -alkylsilylamide, Alkylamine, Alkylendiamine, gegebenenfalls N-alkylierte, gegebenenfalls ungesättigte, Cycloalkylamine, basische Heterocyclen, Ammoniumhydroxide sowie carbocyclische Amine. Beispielhaft seien Natrium-hydroxid, -hydrid, -amid, -methanolat, -carbonat, Kalium-tert.-butanolat, -carbonat, Lithiumdiisopropylamid, Kalium-bis-(trimethylsilyl)-amid, Calciumhydrid, Triethylamin, Triethylendiamin, Cyclohexylamin, N-Cyclohexyl-N,N-dimethyl-amin, N,N-Diethylanilin, Pyridin, 4-(N,N-Dimethylamino)-pyridin, N-Methylmorpholin, Benzyl-trimethyl-ammoniumhydroxid sowie 1,5-Diazabicyclo[5.4.0]undec-5-en (DBU) genannt.

Die Reaktionspartner können als solche, d. h. ohne Zusatz eines Lösungs- oder Verdünnungsmittels, z. B. in der Schmelze, miteinander umgesetzt werden. Zumeist ist jedoch der Zusatz eines inerten Lösungs- oder Verdünnungsmittels oder eines Gemisches derselben vorteilhaft. Als Beispiele für solche Lösungs- oder Verdünnungsmittel seien genannt: aromatische, aliphatische und alicyclische Kohlenwasserstoffe und Halogenkohlenwasserstoffe, wie Benzol, Toluol, Xylol, Chlorbenzol, Brombenzol, Petrolether, Hexan, Cyclohexan, Dichlormethan, Trichlormethan, Dichlorethan oder Trichlorethan; Ester, wie Essigsäureethylester; Ether, wie Diethylether, tert.-Butylmethylether, Ethylenglykolmonomethylether, Ethylenglykolmonoethylether, Ethylenglykoldimethylether, Tetrahydrofuran oder Dioxan; Ketone, wie Aceton, Methylethylketon oder Methylisobutylketon; Alkohole, wie Methanol, Ethanol, Propanol, Butanol, Ethylenglykol oder Glycerin; Amide, wie N,N-Dimethylformamid, N,N-Dimethylacetamid, N-Methylpyrrolidon oder Hexamethylphosphorsäuretriamid; Nitrile, wie Acetonitril; und Sulfoxide, wie Dimethylsulfoxid. Wird die Umsetzung in Gegenwart einer Säure ausgeführt, können auch im Ueberschuss eingesetzte Säuren, z.B. starke organische Carbonsäuren, wie gegebenenfalls, z.B. durch Halogen, substituierte $C_1$-$C_4$-Alkancarbonsäuren, z. B. Ameisensäure, Essigsäure oder Propionsäure, als Lösungs- oder Verdünnungsmittel dienen. Wird die Umsetzung in Gegenwart einer Base ausgeführt, können auch im Ueberschuss eingesetzte Basen, wie Triethylamin, Pyridin, N-Methylmorpholin oder N,N-Diethylanilin, als Lösungs- oder Verdünnungsmittel dienen.

Die Umsetzung erfolgt vorteilhaft in einem Temperaturbereich von etwa -20°C bis etwa +180°C, bevorzugt von etwa 0°C bis etwa +120°C, in vielen Fällen bei der Rückflusstemperatur des verwendeten Lösungsmittels.

In einer bevorzugten Ausführungsform der Variante b) wird eine Verbindung (IV) bei Rückflusstemperatur in einem Ether, vorzugsweise in Dioxan, und in Gegenwart eines Alkylamins, vorzugsweise in Gegenwart von Triethylamin, mit einer Verbindung (V), worin L Alkoxy ist, umgesetzt.

In einer weiteren bevorzugten Ausführungsform der Variante b) wird eine Verbindung (IV) bei Rückflusstemperatur in einem aromatischen Kohlenwasserstoff, vorzugsweise in Toluol, und in Gegenwart einer organischen Carbonsäure, vorzugsweise in Gegenwart von Essigsäure, mit einer Verbindung (V), worin L Hydroxy ist, oder einem Tautomeren davon umgesetzt.

Die Verbindungen der Formel IV sowie deren Salze, sowie die Verbindungen der Formel V und gegebenenfalls deren Tautomere, jeweils in freier Form oder in Salzform, sind bekannt oder können in Analogie zu bekannten Verbindungen hergestellt werden.

Eine verfahrensgemäss oder auf andere Weise erhältliche Verbindung I oder gegebenenfalls ein Tautomeres davon kann in an sich bekannter Weise in eine andere Verbindung I überführt werden, indem man einen oder mehrere Substituenten der Ausgangsverbindung I in üblicher Weise durch (einen) andere(n) erfindungsgemässe(n) Substituenten ersetzt.

Beispielsweise können:

- Hydroxygruppen $R_1$ und/oder $R_2$ zu $C_1$-$C_4$-Alkoxygruppen $R_1$ und/oder $R_2$ alkyliert werden;
- Halogen $R_1$ und/oder $R_2$ in unsubstituierte Positionen des/der betreffenden Phenylringe(s) eingeführt werden; oder
- Mercaptogruppen $R_5$ zu Sulfinyl- oder Sulfonylgruppen $R_5$ oder Sulfinylgruppen $R_5$ zu Sulfonylgruppen $R_5$ oxidiert werden.

Es ist dabei, je nach Wahl der dafür jeweils geeigneten Reaktionsbedingungen und Ausgangsmaterialien, möglich, in einem Reaktionsschritt nur einen Substituenten durch einen anderen erfindungsgemässen Substituenten zu ersetzen, oder es können in demselben Reaktionschritt mehrere Substituenten durch andere erfindungsgemässe Substituenten ersetzt werden.

Salze von Verbindungen der Formel I können in an sich bekannter Weise hergestellt werden. So erhält man beispielsweise Säureadditionssalze von Verbindungen der Formel I durch Behandeln mit einer geeigneten Säure oder einem geeigneten Ionenaustauscherreagens und Salze mit Basen durch Behandeln mit einer geeigneten Base oder einem geeigneten Ionenaustauscherreagens.

Salze von Verbindungen der Formel I können in üblicher Weise in die freien Verbindungen der Formel I überführt werden, Säureadditionssalze z. B. durch Behandeln mit einem geeigneten basischen Mittel oder einem geeigneten Ionenaustauscherreagens und Salze mit Basen z. B. durch Behandeln mit einer geeigneten Säure oder einem geeigneten Ionenaustauscherreagens.

Salze von Verbindungen der Formel I können in an sich bekannter Weise in andere Salze von Verbindungen der Formel I umgewandelt werden, Säureadditionssalze beispielsweise in andere Säureadditionssalze, z. B. durch Behandeln eines Salzes einer anorganischen Säure, wie eines Hydrochlorids, mit einem geeigneten Metallsalz, wie einem Natrium-, Barium- oder Silbersalz, einer Säure, z.B. mit Silberacetat, in einem geeigneten Lösungsmittel, in welchem ein sich bildendes anorganisches Salz, z. B. Silberchlorid, unlöslich ist und damit aus dem Reaktionsgemisch ausscheidet.

Je nach Verfahrensweise bzw. Reaktionsbedingungen können die Verbindungen der Formel I mit salzbildenden Eigenschaften in freier Form oder in Form von Salzen erhalten werden.

Die Verbindungen der Formel I und gegebenenfalls ihre Tautomeren, jeweils in freier Form oder in Salzform, können in Form eines der möglichen Isomeren oder als Gemisch derselben, z. B. je nach Anzahl, absoluter und relativer Konfiguration von im Molekül auftretenden asymmetrischen Kohlenstoffatomen und/oder je nach Konfiguration von im Molekül auftretenden nichtaromatischen Doppelbindungen, als reine Isomere, wie Antipoden und/oder Diastereomere, oder als Isomerengemische, wie Enantiomerengemische, z. B. Racemate, Diastereomerengemische oder Racematgemische, vorliegen; die Erfindung betrifft sowohl die reinen Isomeren als auch alle möglichen Isomerengemische und ist vor- und nachstehend jeweils entsprechend zu verstehen, auch wenn stereochemische Einzelheiten nicht in jedem Fall speziell erwähnt werden.

Verfahrensgemäss - je nach Wahl der Ausgangsstoffe und Arbeitsweisen - oder anderweitig erhältliche Diastereomerengemische und Racematgemische von Verbindungen der Formel I und gegebenenfalls ihrer Tautomeren, jeweils in freier Form oder in Salzform, können auf Grund der physikalisch-chemischen Unterschiede der Bestandteile in bekannter Weise in die reinen Diastereomeren oder Racemate aufgetrennt werden, beispielsweise durch fraktionierte Kristallisation, Destillation und/oder Chromatographie.

Entsprechend erhältliche Enantiomerengemische, wie Racemate, lassen sich nach bekannten Methoden in die optischen Antipoden zerlegen, beispielsweise durch Umkristallisation aus einem optisch aktiven Lösungsmittel, durch Chromatographie an chiralen Adsorbentien, z. B. Hochdruckflüssigkeitschromatographie (HPLC) an Acetylcellulose, mit Hilfe von geeigneten Mikroorganismen, durch Spaltung mit spezifischen, immobilisierten Enzymen, über die Bildung von Einschlussverbindungen, z. B. unter Verwendung chiraler Kronenether, wobei nur ein Enantiomeres komplexiert wird, oder durch Überführung in diastereomere Salze, z. B. durch Umsetzung eines basischen Endstoffracemats mit einer optisch aktiven Säure, wie Carbonsäure, z. B. Campher-, Wein- oder Äpfelsäure, oder Sulfonsäure, z. B. Camphersulfonsäure, und Trennung des auf diese Weise erhaltenen Diastereomerengemisches, z. B. auf Grund ihrer verschiedenen Löslichkeiten durch fraktionierte Kristallisation, in die Diastereomeren, aus denen das gewünschte Enantiomere durch Einwirkung geeigneter, z. B. basischer, Mittel freigesetzt werden kann.

Ausser durch Auftrennung entsprechender Isomerengemische können reine Diastereomere bzw. Enantiomere erfindungsgemäss auch durch allgemein bekannte Methoden der diastereoselektiven bzw. enantioselektiven Synthese erhalten werden, z. B. indem man das erfindungsgemässe Verfahren mit Edukten mit entsprechend geeigneter Stereochemie ausführt.

Vorteilhaft isoliert bzw. synthetisiert man jeweils das biologisch wirksamere Isomere, z. B. Enantiomere oder Diastereomere, oder Isomerengemisch, z. B. Enantiomerengemisch oder Diastereomerengemisch, sofern die einzelnen Komponenten unterschiedliche biologische Wirksamkeit besitzen.

Die Verbindungen der Formel I und gegebenenfalls ihre Tautomeren, jeweils in freier Form oder in Salzform, können auch in Form ihrer Hydrate erhalten werden und/oder andere, beispielsweise gegebenenfalls zur Kristallisation von in fester Form vorliegenden Verbindungen verwendete, Lösungsmittel einschliessen.

Die Erfindung betrifft alle diejenigen Ausführungsformen des Verfahrens, nach denen man von einer auf irgendeiner Stufe des Verfahrens als Ausgangs- oder Zwischenprodukt erhältlichen Verbindung ausgeht und alle oder einige der fehlenden Schritte durchführt oder einen Ausgangsstoff in Form eines Derivates bzw. Salzes und/oder seiner Racemate bzw. Antipoden verwendet oder insbesondere unter den Reaktionsbedingungen bildet.

Beim Verfahren der vorliegenden Erfindung werden vorzugsweise solche Ausgangsstoffe und Zwischenprodukte bzw. deren Salze verwendet, welche zu den eingangs als besonders wertvoll geschilderten Verbindungen der Formel I bzw. deren Salzen führen.

Die Erfindung betrifft insbesondere die in den Beispielen H1 bis H3 beschriebenen Herstellungsverfahren.

Erfindungsgemäss für die Herstellung der Verbindungen der Formel I bzw. ihrer Salze verwendete Ausgangsstoffe und Zwischenprodukte oder deren Salze, die neu sind, ihre Verwendung und Verfahren zu ihrer Herstellung bilden ebenfalls einen Gegenstand der Erfindung.

Die erfindungsgemässen Verbindungen der Formel I sind bei günstiger Warmblüter-, Fisch- und Pflanzenverträglichkeit wertvolle Wirkstoffe auf dem Gebiet der Schädlingsbekämpfung, wobei die vorstehend erwähnte Massgabe (A) für diese Wirkstoffe nicht gültig ist. Insbesondere wirken die erfindungsgemässen Wirkstoffe gegen Insekten, wie sie an Nutz- und Zierpflanzen in der Landwirtschaft und im Gartenbau, insbesondere in Baumwoll-, Gemüse- und Obstpflanzungen, und im Forst vorkommen. Die erfindungsgemässen Wirkstoffe eignen sich besonders zur Bekämpfung von Insekten in Obst- und Gemüsekulturen, insbesondere von pflanzenschädigenden Insekten, wie Spodoptera littoralis, Heliothis virescens, Diabrotica balteata und Crocidolomia binotalis. Weitere Anwendungsgebiete der erfindungsgemässen Wirkstoffe sind der Vorrats- und Materialschutz sowie im Hygienesektor insbesondere der Schutz von Haus- und Nutztieren. Die erfindungsgemässen Wirkstoffe sind gegen alle oder einzelne Entwicklungsstadien von normal sensiblen, aber auch von resistenten Arten von Schädlingen wirksam. Dabei kann sich ihre Wirkung z. B. in einer Abtötung der Schädlinge, welche unmittelbar oder erst nach einiger Zeit, beispielsweise bei einer Häutung, eintritt, oder in einer verminderten Eiablage und/oder Schlupfrate zeigen.

Zu den oben erwähnten Schädlingen gehören:

aus der Ordnung Lepidoptera zum Beispiel

Acleris spp., Adoxophyes spp., Aegeria spp., Agrotis spp., Alabama argillaceae, Amylois spp., Anticarsia gemmatalis, Archips spp., Argyrotaenia spp., Autographa spp., Busseola fusca, Cadra cautella, Carposina nipponensis, Chilo spp., Choristoneura spp., Clysia ambiguella, Cnaphalocrocis spp., Cnephasia spp., Cochylis spp., Coleophora spp., Crocidolomia binotalis, Cryptophlebia leucotreta, Cydia spp., Diatraea spp., Diparopsis castanea, Earias spp., Ephestia spp., Eucosma spp., Eupoecilia ambiguella, Euproctis spp., Euxoa spp., Grapholita spp., Hedya nubiferana, Heliothis spp., Hellula undalis, Hyphantria cunea, Keiferia lycopersicella, Leucoptera scitella, Lithocollethis spp., Lobesia botrana, Lymantria spp., Lyonetia spp., Malacosoma spp., Mamestra brassicae, Manduca sexta, Operophtera spp., Ostrinia nubilalis, Pammene spp., Pandemis spp., Panolis flammea, Pectinophora gossypiella, Phthorimaea operculella, Pieris rapae, Pieris spp., Plutella xylostella, Prays spp., Scirpophaga spp., Sesamia spp., Sparganothis spp., Spodoptera spp., Synanthedon spp., Thaumetopoea spp., Tortrix spp., Trichoplusia ni und Yponomeuta spp.;

aus der Ordnung Coleoptera zum Beispiel

Agriotes spp., Anthonomus spp., Atomaria linearis, Chaetocnema tibialis, Cosmopolites spp., Curculio spp., Dermestes spp., Diabrotica spp., Epilachna spp., Eremnus spp., Leptinotarsa decemlineata, Lissorhoptrus spp., Melolontha spp., Orycaephilus spp., Otiorhynchus spp., Phlyctinus spp., Popillia spp., Psylliodes spp., Rhizopertha spp., Scarabeidae, Sitophilus spp., Sitotroga spp., Tenebrio spp., Tribolium spp. und Trogoderma spp.;

aus der Ordnung Orthoptera zum Beispiel

Blatta spp., Blattella spp., Gryllotalpa spp., Leucophaea maderae, Locusta spp., Periplaneta spp. und Schistocerca spp.;

aus der Ordnung Isoptera zum Beispiel

Reticulitermes spp.;

aus der Ordnung Psocoptera zum Beispiel

Liposcelis spp.;

aus der Ordnung Anoplura zum Beispiel

Haematopinus spp., Linognathus spp., Pediculus spp., Pemphigus spp. und Phylloxera spp.;

aus der Ordnung Mallophaga zum Beispiel

Damalinea spp. und Trichodectes spp.;

aus der Ordnung Thysanoptera zum Beispiel
Frankliniella spp., Hercinothrips spp., Taeniothrips spp., Thrips palmi, Thrips tabaci und Scirtothrips aurantii;
aus der Ordnung Heteroptera zum Beispiel
Cimex spp., Distantiella theobroma, Dysdercus spp., Euchistus spp. Eurygaster spp. Leptocorisa spp., Nezara spp., Piesma spp., Rhodnius spp., Sahlbergella singularis, Scotinophara spp. und Triatoma spp.;
aus der Ordnung Homoptera zum Beispiel
Aleurothrixus floccosus, Aleyrodes brassicae, Aonidiella spp., Aphididae, Aphis spp., Aspidiotus spp., Bemisia tabaci, Ceroplaster spp., Chrysomphalus aonidium, Chrysomphalus dictyospermi, Coccus hesperidum, Empoasca spp., Eriosoma larigerum, Erythroneura spp., Gascardia spp., Laodelphax spp., Lecanium corni, Lepidosaphes spp., Macrosiphus spp., Myzus spp., Nephotettix spp., Nilaparvata spp., Paratoria spp., Pemphigus spp., Planococcus spp., Pseudaulacaspis spp., Pseudococcus spp., Psylla spp., Pulvinaria aethiopica, Quadraspidiotus spp., Rhopalosiphum spp., Saissetia spp., Scaphoideus spp., Schizaphis spp., Sitobion spp., Trialeurodes vaporariorum, Trioza erytreae und Unaspis citri;
aus der Ordnung Hymenoptera zum Beispiel
Acromyrmex, Atta spp., Cephus spp., Diprion spp., Diprionidae, Gilpinia polytoma, Hoplocampa spp., Lasius spp., Monomorium pharaonis, Neodiprion spp., Solenopsis spp. und Vespa spp.;
aus der Ordnung Diptera zum Beispiel
Aedes spp., Antherigona soccata, Bibio hortulanus, Calliphora erythrocephala, Ceratitis spp., Chrysomyia spp., Culex spp., Cuterebra spp., Dacus spp., Drosophila melanogaster, Fannia spp., Gastrophilus spp., Glossina spp., Hypoderma spp., Hyppobosca spp., Liriomyza spp., Lucilia spp., Melanagromyza spp., Musca spp., Oestrus spp., Orseolia spp., Oscinella frit, Pegomyia hyoscyami, Phorbia spp., Rhagoletis pomonella, Sciara spp., Stomoxys spp., Tabanus spp., Tannia spp. und Tipula spp.;
aus der Ordnung Siphonaptera zum Beispiel
Ceratophyllus spp. und Xenopsylla cheopis und
aus der Ordnung Thysanura zum Beispiel
Lepisma saccharina.

Die gute pestizide Wirkung der erfindungsgemässen Wirkstoffe entspricht einer Abtötungsrate (Mortalität) von mindestens 50-60 % der erwähnten Schädlinge.

Die Wirkung der erfindungsgemässen Wirkstoffe und der sie enthaltenden Mittel lässt sich durch Zusatz von anderen Insektiziden wesentlich verbreitern und an gegebene Umstände anpassen. Als Zusätze kommen zum Beispiel Vertreter der folgenden Wirkstoffklassen in Betracht: Organische Phosphorverbindungen, Nitrophenole und Derivate, Formamidine, Harnstoffe, Carbamate, Pyrethroide, chlorierte Kohlenwasserstoffe und Bacillus thuringiensis-Präparate.

Die erfindungsgemässen Wirkstoffe werden in unveränderter Form oder vorzugsweise zusammen mit den in der Formulierungstechnik üblichen Hilfsmitteln eingesetzt und können daher beispielsweise zu emulgierbaren Konzentraten, direkt versprüh- oder verdünnbaren Lösungen, verdünnten Emulsionen, Spritzpulvern, löslichen Pulvern, Stäubemitteln und Granulaten, auch zu Verkapselungen in polymeren Stoffen, in bekannter Weise verarbeitet werden. Die Anwendungsverfahren, wie Versprühen, Vernebeln, Verstäuben, Verstreuen oder Giessen, werden ebenso wie die Mittel den angestrebten Zielen und den gegebenen Verhältnissen entsprechend gewählt.

Die Formulierungen, das heisst die den erfindungsgemässen Wirkstoff, beziehungsweise eine Kombination dieses Wirkstoffs mit anderen Insektiziden, und gegebenenfalls feste oder flüssige Hilfsstoffe enthaltenden Mittel, Zubereitungen oder Zusammensetzungen, werden in bekannter Weise hergestellt, zum Beispiel durch inniges Vermischen und/oder Vermahlen des Wirkstoffs mit den Hilfsstoffen, wie Streckmitteln, z. B. Lösungsmitteln oder festen Trägerstoffen, oder wie oberflächenaktiven Verbindungen (Tensiden).

Als Lösungsmittel können in Frage kommen: Aromatische Kohlenwasserstoffe, bevorzugt die Fraktionen $C_8$ bis $C_{12}$ von Alkylbenzolen, wie Xylolgemische oder alkylierte Naphthaline, aliphatische oder cycloaliphatische Kohlenwasserstoffe, wie Cyclohexan, Paraffine oder Tetrahydronaphthalin, Alkohole wie Ethanol, Propanol oder Butanol, Glykole sowie deren Ether und Ester, wie Propylenglykol, Dipropylenglykolether, Ethylenglykol, Ethylenglykolmonomethyl- oder -ethylether, Ketone, wie Cyclohexanon, Isophoron oder Diacetonalkohol, stark polare Lösungsmittel, wie N-Methyl-2-pyrrolidon, Dimethylsulfoxid oder N,N-Dimethylformamid, Wasser sowie gegebenenfalls epoxidierte Pflanzenöle, wie gegebenenfalls epoxidiertes Raps-, Rizinus-, Kokosnuss- oder Sojaöl; gegebenenfalls auch Silikonöle.

Als feste Trägerstoffe, beispielsweise für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäuren oder hochdisperse saugfähige Polymerisate zugesetzt werden. Als gekörnte, adsorptive Granulatträger kommen poröse Typen, wie Bimsstein, Ziegelbruch, Sepiolit oder Bentonit, als nicht sorptive Trägermaterialien Calcit oder Sand in Frage. Darüberhinaus kann eine Vielzahl von granulierten Materialien anorganischer oder organischer Natur, insbesondere Dolomit oder zerkleinerte Pflanzenrückstände, verwendet werden.

Als oberflächenaktive Verbindungen kommen je nach Art des zu formulierenden erfindungsgemässen Wirkstoffs oder der Kombination dieses Wirkstoffs mit anderen Insektiziden nichtionische, kationische und-/oder anionische Tenside mit guten Emulgier-, Dispergier- und Netzeigenschaften in Betracht. Unter Tensiden sind auch Tensidgemische zu verstehen.

Als nichtionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können. Weiterhin geeignet sind die wasserlöslichen 20 bis 250 Ethylenglykolethergruppen und 10 bis 100 Propylenglykolethergruppen enthaltenden Polyethylenoxid-Addukte an Polypropylenglykol, Ethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Ethylenglykol-Einheiten. Als Beispiele nichtionischer Tenside seien Nonylphenolpolyethoxyethanole, Ricinusölpolyglykolether, Polypropylen-Polyethylenoxid-Addukte, Tributylphenoxypolyethoxyethanol, Polyethylenglykol und Octylphenoxypolyethoxyethanol erwähnt. Ferner kommen auch Fettsäureester von Polyoxyethylensorbitan, wie das Polyoxyethylensorbitan-trioleat, in Betracht.

Bei den kationischen Tensiden handelt es sich vor allem um quarternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Ethylsulfate vor; Beispiele sind das Stearyltrimethylammoniumchlorid oder das Benzyl-di-(2-chlorethyl)-ethylammoniumbromid.

Geeignete anionische Tenside können sowohl sogenannte wasserlösliche Seifen als auch wasserlösliche synthetische oberflächenaktive Verbindungen sein. Als Seifen eignen sich die Alkali-, Erdalkali- oder gegebenenfalls substituierten Ammoniumsalze von höheren Fettsäuren ($C_{10}$-$C_{22}$), wie die Natrium- oder Kalium-Salze der Oel- oder Stearinsäure oder von natürlichen Fettsäuregemischen, die beispielsweise aus Kokosnuss- oder Tallöl gewonnen werden können; ferner sind auch die Fettsäuremethyl-taurin-salze zu erwähnen. Häufiger werden jedoch sogenannte synthetische Tenside verwendet, insbesondere Fettsulfonate, Fettsulfate, sulfonierte Benzimidazolderivate oder Alkylarylsulfonate. Die Fettsulfonate oder -sulfate liegen in der Regel als Alkali-, Erdalkali- oder gegebenenfalls substituierte Ammoniumsalze vor und weisen im allgemeinen einen Alkylrest mit 8 bis 22 C-Atomen auf, wobei Alkyl auch den Alkylteil von Acylresten einschliesst; beispielhaft genannt seien das Natrium- oder Calcium-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Ethylenoxid-Addukten. Die sulfonierten Benzimidazolderivate enthalten vorzugsweise 2 Sulfonsäuregruppen und einen Fettsäurerest mit etwa 8-22 C-Atomen. Alkylarylsulfonate sind zum Beispiel die Natrium-, Calcium- oder Triethanolammoniumsalze der Dodecylbenzolsulfonsäure, der Dibutylnaphthalinsulfonsäure oder eines Naphthalinsulfonsäure-Formaldehyd-Kondensationsproduktes. Ferner kommen auch entsprechende Phosphate, wie Salze des Phosphorsäureesters eines p-Nonylphenol-(4-14)-Ethylenoxid-Adduktes oder Phospholipide, in Frage.

Die vorstehend aufgeführten Tenside sind nur als Beispiele anzusehen; in der einschlägigen Literatur werden viele weitere in der Formulierungstechnik gebräuchliche und erfindungsgemäss geeignete Tenside beschrieben.

Die pestiziden Zubereitungen enthalten in der Regel 0,1 bis 99%, insbesondere 0,1 bis 95%, an dem erfindungsgemässen Wirkstoff oder an der Kombination dieses Wirkstoffs mit anderen Insektiziden und 1 bis 99,9%, insbesondere 5 bis 99,9%, eines festen oder flüssigen Hilfsstoffes, wobei in der Regel 0 bis 25%, insbesondere 0,1 bis 20%, der Zubereitungen Tenside sein können (% bedeutet jeweils Gewichtsprozent). Während als Handelsware eher konzentrierte Mittel bevorzugt werden, verwendet der Endverbraucher in der Regel verdünnte Zubereitungen, die wesentlich geringere Wirkstoffkonzentrationen aufweisen. Typische Anwendungskonzentrationen liegen zwischen 0,1 und 1000 ppm, vorzugsweise zwischen 0,1 und 500 ppm Wirkstoff. Die Aufwandmengen pro Hektar betragen im allgemeinen 1 bis 1000 g Wirkstoff pro Hektar, vorzugsweise 25 bis 500 g/ha. Insbesondere setzen sich bevorzugte Formulierungen folgendermassen zusammen (% = Gewichtsprozent):

<u>Emulgierbare Konzentrate:</u>

| | |
|---|---|
| Wirkstoff: | 1 bis 90 %, bevorzugt 5 bis 20 % |
| Tensid: | 1 bis 30 %, vorzugsweise 10 bis 20 % |

flüssiger Trägerstoff: 5 bis 94 %, vorzugsweise 70 bis 85 %

Stäube:

| | |
|---|---|
| Wirkstoff: | 0,1 bis 10 %, vorzugsweise 0,1 bis 1 % |
| fester Trägerstoff: | 99,9 bis 90 %, vorzugsweise 99,9 bis 99 % |

Suspensions-Konzentrate:

| | |
|---|---|
| Wirkstoff: | 5 bis 75 %, vorzugsweise 10 bis 50 % |
| Wasser: | 94 bis 24 %, vorzugsweise 88 bis 30 % |
| Tensid: | 1 bis 40 %, vorzugsweise 2 bis 30 % |

Benetzbare Pulver:

| | |
|---|---|
| Wirkstoff: | 0,5 bis 90 %, vorzugsweise 1 bis 80 % |
| Tensid: | 0,5 bis 20 %, vorzugsweise 1 bis 15 % |
| fester Trägerstoff: | 5 bis 95 %, vorzugsweise 15 bis 90 % |

Granulate:

| | |
|---|---|
| Wirkstoff: | 0,5 bis 30 %, vorzugsweise 3 bis 15 % |
| fester Trägerstoff: | 99,5 bis 70 %, vorzugsweise 97 bis 85 % |

Die Zubereitungen können auch weitere Hilfsstoffe, wie Stabilisatoren, z. B. gegebenenfalls epoxidierte Pflanzenöle (z. B. epoxidiertes Kokosnussöl, Rapsöl oder Sojaöl), Entschäumer, z. B. Silikonöl, Konservierungsmittel, Viskositätsregulatoren, Bindemittel und/oder Haftmittel, sowie Dünger oder andere Wirkstoffe zur Erzielung spezieller Effekte enthalten.

Die folgenden Beispiele dienen der Erläuterung der Erfindung. Sie schränken die Erfindung nicht ein. Temperaturen sind in Grad Celsius angegeben.

Herstellungsbeispiele

Beispiel H1:

1-(4-Chlorphenyl)-5-ethansulfonyl-2,3,5-triaza-1-(4-trifluormethansulfonyloxyphenyl)-penta-1,4-dien

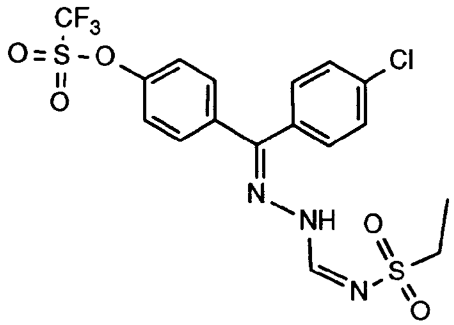

bzw. 1-(4-Chlorphenyl)-5-ethansulfonyl-2,3,5-triaza-1-(4-trifluormethansulfonyloxyphenyl)-penta-1,3-dien

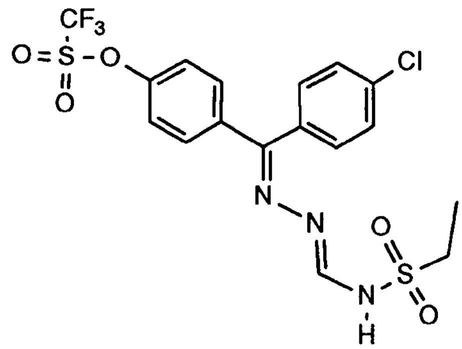

Zu einer Lösung von 15,2 g 4-Chlor-4'-trifluormethansulfonyloxy-benzophenonhydrazon in 150 ml 1,4-Dioxan werden 15,7 g 1-Aza-1-ethansulfonyl-3-oxa-pent-1-en und 28 ml Triethylamin zugegeben. Das Gemisch wird 17 Stunden unter Rückfluss gerührt, auf Raumtemperatur abgekühlt und am Rotationsverdampfer im Vakuum eingedampft. Der Rückstand wird an Silicagel mit Dichlormethan als Laufmittel chromatographiert. Man erhält so die Titelverbindung in Form eines Isomerengemisches, das bei 121 bis 129°C schmilzt (Verbindung Nr. 1.2).

Beispiel H2:

1-(4-Chlorphenyl)-2,3-diaza-4-methyl-6-oxo-1-(4-trifluormethansulfonyloxyphenyl)-hepta-1,4-dien

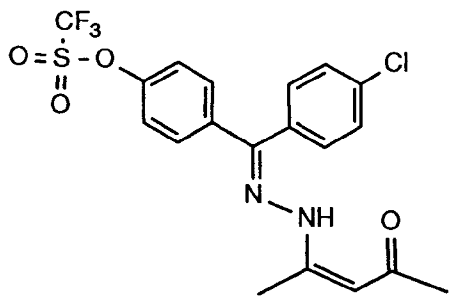

bzw. 1-(4-Chlorphenyl)-2,3-diaza-4-methyl-6-oxo-1-(4-trifluormethansulfonyloxyphenyl)-hepta-1,3-dien

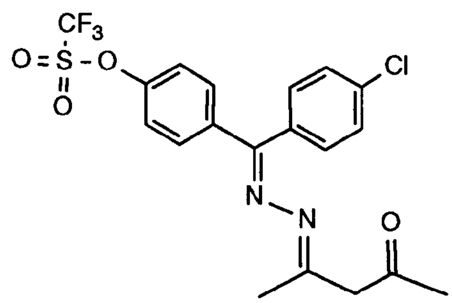

Zu einer Lösung von 3,8 g 4-Chlor-4'-trifluormethansulfonyloxy-benzophenonhydrazon in 50 ml Toluol werden 1,2 g Pentan-2,4-dion und 0,5 ml Eisessig zugegeben. Das Reaktionsgemisch wird 12 Stunden unter Rückfluss gerührt, auf Raumtemperatur abgekühlt und am Rotationsverdampfer im Vakuum eingedampft. Der Rückstand wird an Silicagel bei einem Druck von 35 Bar mit Ethylacetat/Hexan (1:20) als Laufmittel chromatographiert. Man erhält so die Titelverbindung in Form eines öligen Isomerengemisches (Verbindung Nr. 3.5).

Beispiel H3:

In analoger Weise wie in den Beispielen H1 und H2 beschrieben können auch die anderen in den Tabellen

1 bis 5 aufgeführten Verbindungen der Formel I bzw. gegebenenfalls deren Tautomere hergestellt werden. Sofern Isomerengemische in zwei Komponenten aufgetrennt wurden, sind die einzelnen Komponenten mit "A" bzw. "B" bezeichnet. In der Spalte "Smp." der Tabellen bezeichnen die angegebenen Temperaturen jeweils den Schmelzpunkt der betreffenden Verbindung.

Tabelle 1

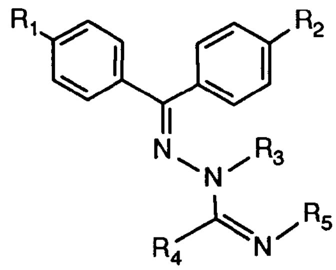

| Verb. Nr. | $R_1$ | $R_2$ | $R_3$ | $R_4$ | $R_5$ | Smp. °C |
|---|---|---|---|---|---|---|
| 1.1 | $-OSO_2CF_3$ | Cl | H | H | $-SO_2CH_3$ | 121-129 |
| 1.2 | $-OSO_2CF_3$ | Cl | H | H | $-SO_2CH_2CH_3$ | 75-80 |
| 1.3 | $-OSO_2CF_3$ | Cl | H | H | $-SO_2n-C_4H_9$ | Amorph |
| 1.4 | $-OSO_2CF_3$ | Cl | H | H | $-SO_2C_6H_5$ | 176-178 |
| 1.5 | $-OSO_2CF_3$ | Cl | H | H | $-SO_2C_6H_4-4-CH_3$ | 155-160 |
| 1.6 | $-OSO_2CF_3$ | Cl | H | H | $-SO_2C_6H_4-4-Cl$ | Amorph |
| 1.7 | $-OSO_2CF_3$ | Cl | H | H | $-SO_2C_6H_4-4-I$ | 98 |
| 1.8 | $-OSO_2CF_3$ | Cl | H | H | $-SO_2C_6H_4-4-OCH_3$ | Amorph |
| 1.9 | $-OSO_2CH_3$ | Cl | H | H | $-SO_2CH_3$ | Amorph |
| 1.10 | $-OSO_2CH_3$ | Cl | H | H | $-SO_2CH_2CH_3$ | 85-105 |
| 1.11 | $-OSO_2CH_3$ | Cl | H | H | $-SO_2C_6H_4-4-CH_3$ | 47-51 |
| 1.12 | -H | H | H | H | $-SO_2CH_2CH_3$ | 145 |
| 1.13A | -OH | Cl | H | H | $-SO_2CH_2CH_3$ | 86-108 |
| 1.13B | -OH | Cl | H | H | $-SO_2CH_2CH_3$ | 162-170 |
| 1.14A | -OH | Cl | H | H | $-SO_2CH_3$ | 177-185 |
| 1.14B | -OH | Cl | H | H | $-SO_2CH_3$ | 168-173 |
| 1.15 | $-OSO_2CH_3$ | Cl | $CH_3$ | H | $-SO_2CH_2CH_3$ | Schaum |
| 1.16 | $-OSO_2CF_3$ | Cl | $CH_3$ | H | $-SO_2CH_2CH_3$ | Harz |
| 1.17 | $-OSO_2CF_3$ | Cl | H | $CH_3$ | $-SO_2CH_3$ | Harz |
| 1.18 | $-OSO_2CF_3$ | Cl | H | $CH_3$ | $-SO_2CH_2CH_3$ | Harz |
| 1.19 | $-OSO_2CF_3$ | Cl | H | H | $-SO_2CH_2Cl$ | Amorph |
| 1.20 | $-OSO_2CF_3$ | Cl | H | H | $-SO_2CH_2Br$ | |
| 1.21 | $-OSO_2CF_3$ | F | H | H | $-SO_2CH_3$ | 168-171 |
| 1.22 | $-OSO_2CF_3$ | F | H | H | $-SO_2CH_2CH_3$ | 65 |
| 1.23 | $-OSO_2CF_3$ | F | H | H | $-SO_2CH_2CH_2CH_3$ | |
| 1.24 | $-OSO_2CF_3$ | F | H | H | $-SO_2CH(CH_3)_2$ | |

Tabelle 1 (Fortsetzung)

| Verb. Nr. | $R_1$ | $R_2$ | $R_3$ | $R_4$ | $R_5$ | Smp. °C |
|---|---|---|---|---|---|---|
| 1.25 | $-OSO_2CF_3$ | Cl | H | H | $-SO_2CH_2CH_2CH_3$ | Harz |
| 1.26 | $-OSO_2CF_3$ | Cl | H | H | $-SO_2CH(CH_3)_2$ | Harz |
| 1.27 | $-OSO_2CF_3$ | Cl | H | H | $-SO_2CH_2CH(CH_3)_2$ | |
| 1.28 | $-OSO_2CF_3$ | Cl | H | H | $-SO_2C(CH_3)_3$ | |
| 1.29 | $-OSO_2CF_3$ | Cl | H | H | $-SO_2CH_2C(CH_3)_3$ | |
| 1.30 | $-OSO_2CF_3$ | Cl | H | H | $-SO_2$-cyclo-$C_3H_5$ | |
| 1.31 | $-OSO_2CF_2CF_2Cl$ | Cl | H | H | $-SO_2CH_3$ | |
| 1.32 | $-OSO_2CF_2CF_2Cl$ | Cl | H | H | $-SO_2CH_2CH_3$ | |
| 1.33 | $-OSO_2CF_2CF_3$ | Cl | H | H | $-SO_2CH_3$ | |
| 1.34 | $-OSO_2CF_2CF_3$ | Cl | H | H | $-SO_2CH_2CH_3$ | |
| 1.35 | $-OSO_2CF_3$ | Cl | H | H | -CN | |
| 1.36 | $-OSO_2CF_3$ | Cl | H | H | $-CO_2CH_3$ | |
| 1.37 | $-OSO_2CF_3$ | Cl | H | H | $-CO_2CH_2CH_3$ | |
| 1.38 | $-OSO_2CH_2Cl$ | Cl | H | H | $-SO_2CH_3$ | 55-66 |
| 1.39 | $-OSO_2CH_2Cl$ | Cl | H | H | $-SO_2CH_2CH_3$ | Schaum |
| 1.40 | $-OSO_2CF_3$ | Cl | H | H | $-SO_2CH_2C_6H_5$ | amorph |
| 1.41 | $-OSO_2CF_3$ | Cl | $CH_3$ | H | $-SO_2CH_3$ | Harz |
| 1.42A | $-OSO_2CF_3$ | F | H | H | $-SO_2C_6H_5$ | 65-67 |
| 1.42B | $-OSO_2CF_3$ | F | H | H | $-SO_2C_6H_5$ | 75-78 |
| 1.43 | $-OSO_2CF_3$ | F | H | H | $-SO_2CH_2C_6H_5$ | 74-81 |
| 1.44 | $-OSO_2CF_3$ | F | H | $CH_3$ | $-SO_2C_2H_5$ | Oel |
| 1.45 | $-OSO_2CF_3$ | F | $CH_3$ | H | $-SO_2C_2H_5$ | Oel |
| 1.46 | $-NO_2$ | Cl | H | H | $-SO_2C_2H_5$ | 88-95 |
| 1.47 | $-N(C_2H_5)SO_2CF_3$ | Cl | H | H | $-SO_2C_2H_5$ | amorph |
| 1.48A | $-OSO_2CF_3$ | Br | H | H | $-SO_2CH_2C_6H_5$ | Wachs |
| 1.48B | $-OSO_2CF_3$ | Br | H | H | $-SO_2CH_2C_6H_5$ | Wachs |
| 1.49 | $-OSO_2CF_3$ | $CH_3$ | H | H | $-SO_2C_2H_5$ | 95-97 |
| 1.50 | $-OSO_2CF_3$ | $CH_3$ | H | H | $-SO_2CH_2C_6H_5$ | 122-140 |
| 1.51 | $-OSO_2CF_3$ | $CH_3$ | H | H | $-SO_2C_6H_5$ | 171-175 |
| 1.52 | $-OSO_2CF_3$ | t-$C_4H_9$ | H | H | $-SO_2CH_2C_6H_5$ | 122-132 |
| 1.53 | $-OSO_2CF_3$ | t-$C_4H_9$ | H | H | $-SO_2C_6H_5$ | 153-155 |
| 1.54 | $-OSO_2CF_3$ | t-$C_4H_9$ | H | $CH_3$ | $-SO_2C_2H_5$ | Oel |
| 1.55 | $-OSO_2CF_3$ | t-$C_4H_9$ | H | H | $-SO_2C_2H_5$ | 172-176 |
| 1.56 | $-OC_6H_5$ | Cl | H | H | $-SO_2CH_2C_6H$ | Wachs |
| 1.57 | $-OC_6H_5$ | Cl | H | H | $-SO_2C_2H_5$ | Wachs |

Tabelle 1 (Fortsetzung)

| Verb. Nr. | $R_1$ | $R_2$ | $R_3$ | $R_4$ | $R_5$ | Smp. °C |
|---|---|---|---|---|---|---|
| 1.58 | $-OC_6H_5$ | Cl | H | H | $-SO_2C_6H_5$ | Wachs |
| 1.59 | $-OCF_2CHF_2$ | F | H | H | $-SO_2N(CH_3)_2$ | Harz |
| 1.60 | $-OCF_2CHF_2$ | Cl | H | H | $-SO_2C_2H_5$ | Schaum |
| 1.61 | $-OSO_2CH_3$ | Br | H | H | $-SO_2C_2H_5$ | Schaum |
| 1.62 | $-OSO_2C_6H_5$ | Cl | H | H | $-SO_2CH_2C_6H_5$ | Wachs |
| 1.63 | $-OSO_2C_6H_5$ | Cl | H | H | $-SO_2C_6H_5$ | 88-100 |
| 1.64 | $-OSO_2C_6H_5$ | Cl | H | H | $-SO_2C_2H_5$ | 95-103 |

Tabelle 2

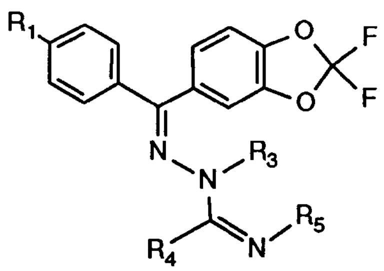

| Verb. Nr. | $R_1$ | $R_3$ | $R_4$ | $R_5$ | Smp. |
|---|---|---|---|---|---|
| 2.1 | Cl | H | H | $-SO_2CH_3$ | 181-183,5° |
| 2.2 | Cl | H | H | $-SO_2CH_2CH_3$ | 65-70° |
| 2.3 | Cl | H | H | $-SO_2CH_2CH_2CH_3$ | |
| 2.4 | Cl | H | H | $-SO_2(CH_2)_3CH_3$ | |
| 2.5 | Cl | H | H | $-SO_2CH(CH_3)_2$ | |
| 2.6 | Cl | H | H | $-SO_2CH_2CH(CH_3)_2$ | |

Tabelle 3

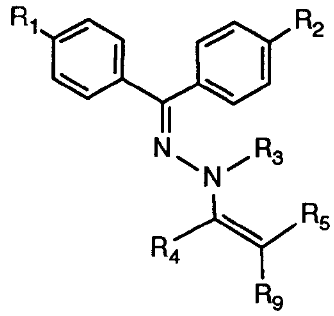

| Verb.Nr. | $R_1$ | $R_2$ | $R_3$ | $R_4$ | $R_5$ | $R_9$ | Smp.°C |
|---|---|---|---|---|---|---|---|
| 3.1A | $-OSO_2CH_3$ | Cl | H | $CH_3$ | $-C(=O)CH_3$ | H | 124-125 |
| 3.1B | $-OSO_2CH_3$ | Cl | H | $CH_3$ | $-C(=O)CH_3$ | H | 143-144 |
| 3.2 | $-OSO_2CH_3$ | Cl | H | $CF_3$ | $-C(=O)CH_3$ | H | Schaum |
| 3.3 | $-OSO_2CH_3$ | Cl | H | $CF_3$ | $-C(=O)CF_3$ | H | 126-127,5 |
| 3.4 | $-OSO_2CH_3$ | Cl | H | $C_6H_5$ | $-C(=O)CH_3$ | H | 118-122 |

Tabelle 3 (Fortsetzung)

| Verb.Nr. | $R_1$ | $R_2$ | $R_3$ | $R_4$ | $R_5$ | $R_9$ | Smp.°C |
|---|---|---|---|---|---|---|---|
| 3.5 | $-OSO_2CF_3$ | Cl | H | $CH_3$ | $-C(=O)CH_3$ | H | Oel |
| 3.5A | $-OSO_2CF_3$ | Cl | H | $CH_3$ | $-C(=O)CH_3$ | H | 118-120 |
| 3.5B | $-OSO_2CF_3$ | Cl | H | $CH_3$ | $-C(=O)CH_3$ | H | Oel |
| 3.6 | $-OSO_2CF_3$ | Cl | H | $CF_3$ | $-C(=O)CH_3$ | H | Harz |
| 3.7 | $-OSO_2CF_3$ | Cl | H | $CF_3$ | $-C(=O)CF_3$ | H | Harz |
| 3.8 | $-OSO_2CF_3$ | Cl | H | $C_6H_5$ | $-C(=O)CH_3$ | H | 97-102 |
| 3.9 | -OH | Cl | H | $CH_3$ | $-C(=O)CH_3$ | H | 194-197 |
| 3.10 | $-OSO_2CF_3$ | Cl | H | H | -CN | -CN | 182-186 |
| 3.11 | $-OSO_2CF_3$ | Cl | H | H | -CN | $-C(=O)OC_2H_5$ | 152-154° |
| 3.12 | $-OSO_2CF_3$ | Cl | H | H | $-C(=O)OC_2H_5$ | $-C(=O)OC_2H_5$ | 85-87° |
| 3.13A | $-OSO_2CF_3$ | Cl | H | $CH_3$ | $-C(=O)OCH_3$ | H | 130-134° |
| 3.13B | $-OSO_2CF_3$ | Cl | H | $CH_3$ | $-C(=O)OCH_3$ | H | 90-92° |
| 3.14 | $-OSO_2CF_3$ | Cl | H | $CH_3$ | $-C(=O)OC_2H_5$ | H | 86-90° |
| 3.15 | $-OSO_2CF_3$ | Cl | H | $CH_3$ | -C(=O)O-t-Bu | H | 109-117° |
| 3.16 | $-OSO_2CH_3$ | Cl | H | $CH_3$ | -C(=O)O-t-Bu | H | 116-120° |
| 3.17A | $-OSO_2CF_3$ | $CH_3$ | H | $CH_3$ | $-C(=O)CH_3$ | H | 114-117 |
| 3.17B | $-OSO_2CF_3$ | $CH_3$ | H | $CH_3$ | $-C(=O)CH_3$ | H | Wachs |
| 3.18 | $-OSO_2CF_3$ | $t-C_4H_9$ | H | $CH_3$ | $-C(=O)CH_3$ | H | Oel |
| 3.19A | $-OSO_2CF_3$ | F | H | $CH_3$ | $-C(=O)CH_3$ | H | 95-97 |
| 3.19B | $-OSO_2CF_3$ | F | H | $CH_3$ | $-C(=O)CH_3$ | H | 96-97 |
| 3.20A | $-OSO_2CF_3$ | F | H | $C_2H_5$ | $-C(=O)C_2H_5$ | H | 126-127 |
| 3.20B | $-OSO_2CF_3$ | F | H | $C_2H_5$ | $-C(=O)C_2H_5$ | H | Harz |
| 3.21A | $-OSO_2CF_3$ | F | H | $i-C_3H_7$ | $-C(=O)C_3H_9-i$ | H | 123-124 |
| 3.21B | $-OSO_2CF_3$ | F | H | $i-C_3H_7$ | $-C(=O)C_3H_9-i$ | H | 75-77 |
| 3.22A | $-OSO_2CF_3$ | Br | H | $CH_3$ | $-C(=O)CH_3$ | H | 120-122 |
| 3.22B | $-OSO_2CF_3$ | Br | H | $CH_3$ | $-C(=O)CH_3$ | H | 104-107 |
| 3.23A | $-OSO_2CF_3$ | $CH_3$ | H | $CH_3$ | $-C(=O)CH_3$ | H | 114-117 |
| 3.23B | $-OSO_2CF_3$ | $CH_3$ | H | $CH_3$ | $-C(=O)CH_3$ | H | Wachs |
| 3.24 | $-OSO_2CF_3$ | $t-C_4H_9$ | H | $CH_3$ | $-C(=O)CH_3$ | H | Oel |

Tabelle 4

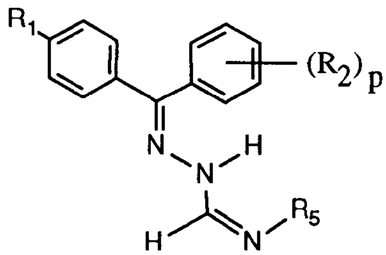

| Verb. Nr. | $R_1$ | $R_2$ | $R_5$ | Smp. °C |
|---|---|---|---|---|
| 4.1A | $-OSO_2CF_3$ | 3-Cl | $-SO_2C_2H_5$ | Oel |
| 4.1B | $-OSO_2CF_3$ | 3-Cl | $-SO_2C_2H_5$ | 80-89 |
| 4.2A | $-OSO_2CF_3$ | 3-Cl | $-SO_2CH_2C_6H_5$ | Wachs |
| 4.2B | $-OSO_2CF_3$ | 3-Cl | $-SO_2CH_2C_6H_5$ | 100-109 |
| 4.3 | $-OSO_2CF_3$ | 3-Cl | $-SO_2C_6H_5$ | 104-116 |
| 4.4 | $-OSO_2CF_3$ | $3,4-Cl_2$ | $-SO_2C_6H_5$ | Wachs |
| 4.5 | $-OSO_2CF_3$ | $3,4-Cl_2$ | $-SO_2C_2H_5$ | Oel |
| 4.6 | $-OSO_2CF_3$ | $2,4-Cl_2$ | $-SO_2C_2H_5$ | Wachs |
| 4.12 | $-OSO_2CF_3$ | $2,4-Cl_2$ | $-SO_2C_6H_5$ | Wachs |
| 4.13 | $-OSO_2CF_3$ | 2-Cl | $-SO_2CH_2C_6H_5$ | Wachs |
| 4.14 | $-OSO_2CF_3$ | 2-Cl | $-SO_2C_6H_5$ | Wachs |
| 4.15 | $-OSO_2CF_3$ | 2-Cl | $-SO_2C_2H_5$ | Wachs |

Tabelle 5

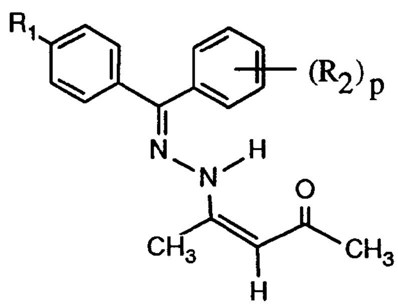

| Verb.Nr. | $R_1$ | $R_2$ | Smp.°C |
|---|---|---|---|
| 5.1A | $-OSO_2CF_3$ | 3-Cl | 91-93 |
| 5.1B | $-OSO_2CF_3$ | 3-Cl | 94-97 |
| 5.2 | $-OSO_2CF_3$ | 3,4-$Cl_2$ | 92-94 |

Formulierungsbeispiele (% = Gewichtsprozent)

| Beispiel F1: Emulsions-Konzentrate | a) | b) | c) |
|---|---|---|---|
| Wirkstoff Nr. 1.2 | 25 % | 40 % | 50 % |
| Ca-Dodecylbenzolsulfonat | 5 % | 8 % | 6 % |
| Ricinusölpolyethylenglykolether (36 Mol EO) | 5 % | - | - |
| Tributylphenolpolyethylenglykolether (30 Mol EO) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

| Beispiel F2: Lösungen | a) | b) | c) | d) |
|---|---|---|---|---|
| Wirkstoff Nr. 1.2 | 80 % | 10 % | 5 % | 95 % |
| Ethylenglykolmonomethylether | 20 % | - | - | - |

| | | | | |
|---|---|---|---|---|
| Polyethylenglykol MG 400 | - | 70 % | - | - |
| N-Methyl-2-pyrrolidon | - | 20 % | - | - |
| Epoxidiertes Kokosnussöl | - | - | 1 % | 5 % |
| Benzin (Siedegrenzen 160-190°C) | - | - | 94 % | - |

Die Lösungen sind zur Anwendung in Form kleinster Tropfen geeignet.

| Beispiel F3: Granulate | a) | b) | c) | d) |
|---|---|---|---|---|
| Wirkstoff Nr. 1.2 | 5 % | 10 % | 8 % | 21 % |
| Kaolin | 94 % | - | 79 % | 54 % |
| Hochdisperse Kieselsäure | 1 % | - | 13 % | 7 % |
| Attapulgit | - | 90 % | - | 18 % |

Der Wirkstoff wird in Dichlormethan gelöst, auf den Träger aufgesprüht und das Lösungsmittel anschliessend im Vakuum abgedampft.

| Beispiel F4: Stäubemittel | a) | b) |
|---|---|---|
| Wirkstoff Nr. 1.2 | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff erhält man gebrauchsfertige Staubemittel.

| Beispiel F5: Spritzpulver | a) | b) | c) |
|---|---|---|---|
| Wirkstoff Nr. 1.2 | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyethylenglykolether (7-8 Mol EO) | - | 2 % | - |
| Hochdisperse Kieselsäure | 5 % | 10 % | 10 % |
| Kaolin | 62 % | 27 % | - |

Der Wirkstoff wird mit den Zusatzstoffen vermischt und in einer geeigneten Mühle gut vermahlen. Man erhält Spritzpulver, die sich mit Wasser zu Suspensionen jeder gewünschten Konzentration verdünnen lassen.

| Beispiel F6: Emulsions-Konzentrat | |
|---|---|
| Wirkstoff Nr. 1.2 | 10 % |
| Octylphenolpolyethylenglykolether (4-5 Mol EO) | 3 % |
| Ca-Dodecylbenzolsulfonat | 3 % |
| Ricinusölpolyglykolether (36 Mol EO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| Beispiel F7: Stäubemittel | a) | b) |
|---|---|---|
| Wirkstoff Nr. 1.2 | 5 % | 8 % |
| Talkum | 95 % | - |
| Kaolin | - | 92 % |

Man erhält anwendungsfertige Stäubemittel, indem der Wirkstoff mit dem Träger vermischt und auf einer geeigneten Mühle vermahlen wird.

| Beispiel F8: Extruder-Granulat | |
|---|---|
| Wirkstoff Nr. 1.2 | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert, granuliert und anschliessend im Luftstrom getrocknet.

| Beispiel F9: Umhüllungs-Granulat | |
|---|---|
| Wirkstoff Nr. 1.2 | 3 % |
| Polyethylenglykol (MG 200) | 3 % |
| Kaolin | 94 % |

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

| Beispiel F10: Suspensions-Konzentrat | |
|---|---|
| Wirkstoff Nr. 1.2 | 40 % |
| Ethylenglykol | 10 % |
| Nonylphenolpolyethylenglykolether (15 Mol EO) | 6 % |
| Na-Ligninsulfonat | 10 % |
| Carboxymethylcellulose | 1 % |
| 37%-ige wässrige Formaldehyd-Lösung | 0,2 % |
| Silikonöl in Form einer 75 %igen wässrigen Emulsion | 0,8 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Biologische Beispiele

Beispiel B1: Wirkung gegen Spodoptera littoralis

Junge Sojapflanzen werden mit einer wässrigen Emulsionsspritzbrühe, die 400 ppm Wirkstoff enthält, besprüht. Nach dem Antrocknen des Spritzbelags werden die Pflanzen mit 10 Raupen des dritten Stadiums von Spodoptera littoralis besiedelt und in einen Plastikbehälter gegeben. 3 Tage später erfolgt die Auswertung. Aus den Vergleichen der Anzahl toter Raupen und des Frassschadens zwischen den behandelten und unbehandelten Pflanzen werden die prozentuale Reduktion der Population und die prozentuale Reduktion des Frassschadens (% Wirkung) bestimmt.
Verbindungen der Tabellen 1 bis 5 zeigen in diesem Test gute Wirkung. Insbesondere zeigen die Verbindungen Nr. 1.1, 1.2, 1.3, 1.5, 1.6, 1.7, 1.8, 1.9, 1.11, 1.16, 1.17, 1.21, 1.22, 1.25, 1.41, 1.42A, 1.42B, 3.5, 3.19A, 3.19B, 3.20A und 3.20B eine Wirkung von mehr als 80%.

Beispiel B2: Wirkung gegen Diabrotica balteata

Maiskeimlinge werden mit einer wässrigen Emulsionsspritzbrühe, die 400 ppm Wirkstoff enthält, besprüht. Nach dem Antrocknen des Spritzbelags werden die Keimlinge mit 10 Larven des zweiten Stadiums von Diabrotica balteata besiedelt und in einen Plastikbehälter gegeben. 6 Tage später erfolgt die Auswertung. Aus dem Vergleich der Anzahl toter Larven zwischen den behandelten und unbehandelten Pflanzen wird die prozentuale Reduktion der Population (% Wirkung) bestimmt.
Verbindungen der Tabellen 1 bis 5 zeigen in diesem Test gute Wirkung. Insbesondere zeigen die Verbindungen Nr. 1.1, 1.2, 1.3, 1.6, 1.7, 1.11, 1.16, 1.17, 1.21, 1.22, 1.25, 1.41, 2.1, 2.2, 3.1 3.19A, 3.19B, 3.20A, 3.20B, 3.22A und 3.22B eine Wirkung von mehr als 80%.

Beispiel B3: Ovizide Wirkung gegen Heliothis virescens

Auf Filterpapier abgelegte Eier von Heliothis virescens werden für kurze Zeit in eine acetonisch-wässrige Testlösung, die 400 ppm Wirkstoff enthält, eingetaucht. Nach dem Antrocknen der Testlösung werden die Eier in Petrischalen inkubiert. Nach 6 Tagen wird der prozentuale Schlupf der Eier im Vergleich zu unbehandelten Kontrollansätzen ausgewertet (% Schlupfreduktion).
Verbindungen der Tabellen 1 bis 5 zeigen in diesem Test gute Wirkung. Insbesondere zeigen die Verbindungen Nr. 1.1, 1.2, 1.3, 1.4, 1.5, 1.6, 1.7, 1.8, 1.9, 1.10, 1.12, 1.16, 1.17, 1.21, 1.22, 1.25, 1.41, 1.42A, 1.42B, 3.1, 3.5, 3.6, 3.19A, 3.19B, 3.20B, 3.22A, 3.22B, 4.4 und 4.5 eine Wirkung von mehr als 80%.

Beispiel B4: Wirkung gegen Heliothis virescens

Junge Sojapflanzen werden mit einer wässrigen Emulsionsspritzbrühe, die 400 ppm Wirkstoff enthält, besprüht. Nach dem Antrocknen des Spritzbelages werden die Pflanzen mit 10 Raupen des ersten Stadiums von Heliothis virescens besiedelt und in einen Plastikbehälter gegeben. 6 Tage später erfolgt die Auswertung. Aus den Vergleichen der Anzahl toter Raupen und des Frassschadens zwischen den behandelten und unbehandelten Pflanzen werden die prozentuale Reduktion der Population und des Frasssschadens (% Wirkung) bestimmt.
Verbindungen der Tabellen 1 bis 5 zeigen in diesem Test gute Wirkung. Insbesondere zeigen die Verbindungen Nr. 1.1, 1.2, 1.3, 1.4, 1.5, 1.6, 1.7, 1.8, 1.9, 1.10, 1.11, 1.16, 1.17, 1.21, 1.22, 1.25, 1.42A, 1.42B, 3.1, 3.19A und 3.19B eine Wirkung von mehr als 80%.

Beispiel B5: Wirkung gegen Crocidolomia binotalis

Junge Kohlpflanzen werden mit einer wässrigen Emulsionsspritzbrühe, die 400 ppm Wirkstoff enthält, besprüht. Nach dem Antrocknen des Spritzbelages werden die Kohlpflanzen mit 10 Raupen des dritten Stadiums von Crocidolmia binotalis besiedelt und in einen Plastikbehälter gegeben. 3 Tage später erfolgt die Auswertung. Aus den Vergleichen der Anzahl toter Raupen und des Frassschadens zwischen den behandelten und unbehandelten Pflanzen werden die prozentuale Reduktion der Population und die prozentuale Reduktion des Frassschadens (% Wirkung) bestimmt.
Verbindungen der Tabellen 1 bis 5 zeigen in diesem Test gute Wirkung. Insbesondere zeigen die Verbindungen Nr. 1.1, 1.2, 1.3, 1.5, 1.6, 1.7, 1.8 und 3.2 eine Wirkung von mehr als 80%.

Beispiel B6: Wirkung gegen Plutella xylostella

Junge Kohlpflanzen werden mit einer wässrigen Emulsionsspritzbrühe, die 400 ppm Wirkstoff enthält, besprüht. Nach dem Antrocknen des Spritzbelags werden die Pflanzen mit 10 Raupen des dritten Stadiums von Plutella xylostella besiedelt und in einen Plastikbehälter gegeben. 3 Tage später erfolgt die Auswertung. Aus den Vergleichen der Anzahl toter Raupen und des Frassschadens zwischen den behandelten und unbehandelten Pflanzen werden die prozentuale Reduktion der Population und die prozentuale Reduktion des Frassschadens (% Wirkung) bestimmt.
Verbindungen der Tabellen 1 bis 5 zeigen in diesem Test gute Wirkung. Insbesondere zeigen die Verbindungen Nr. 1.17, 1.19, 1.20, 1.26, 1.27, 3.5, 3.6, 3.7 und 3.10 eine Wirkung von mehr als 80%.

Beispiel B7: Wirkung gegen Schmeissfliegen Lucilia cuprina

Frisch abgelegte Eier der Schmeissfliegenart Lucilia cuprina werden in kleinen Portionen (30 bis 50 Eier) in Reagenzgläser gegeben, in denen zuvor 4 ml Nährmedium mit 1 ml Testlösung, die 16 ppm des zu prüfenden Wirkstoffes enthält, vermischt worden sind. Nach Beimpfung des Kulturmediums werden die Testgläser mit einem Wattestopfen verschlossen und im Brutschrank bei 30°C 4 Tage lang bebrütet. Im unbehandelten Medium entwickeln sich bis zu diesem Zeitpunkt ca. 1 cm lange Larven (Stadium 3). Ist die Substanz aktiv, so sind die Larven zu diesem Zeitpunkt entweder tot oder deutlich zurückgeblieben. Die Auswertung erfolgt nach 96 Stunden.

Die Verbindungen der Tabellen 1 bis 5 zeigen eine gute Wirkung gegen Lucilia cuprina in diesem Test. Insbesondere die Verbindungen 1.1, 1.2, 1.4, 1.5, 1.7, 1.8, 1.19, 1.40 und 1.41 zeigen eine Wirkung über 80 %.

Beispiel B8: Frasswirkung gegen Ctenocephalides felis (systemisch)

20 Adulte Flöhe der Art Ctenocephalides felis werden in einen flachen runden Käfig gegeben, der auf beiden Seiten mit Gaze verschlossen ist. Auf diesen Käfig wird nun ein Gefäss gestellt, das auf der unteren Seite mit einer Parafilmmembran verschlossen ist. Das Gefäss enthält Blut, dass 50 ppm des Wirkstoffes enthält und konstant auf 37°C erwärmt wird. Die Flöhe nehmen das Blut durch die Membran auf. 24 und 48 Stunden nach Ansatz erfolgt die Auswertung. Aus dem Vergleich der Anzahl toter Flöhe mit behandeltem Blut zu denjenigen mit unbehandeltem Blut wird die prozentuale Reduktion der Population (% Wirkung) bestimmt. 24 Stunden nach Behandlung wird das Blut durch neues ebenfalls behandeltes ersetzt.
Die Verbindung der Tabellen 1 bis 5 zeigen eine gute Wirkung gegen Ctenocephalides felis in diesem Test. Insbesondere die Verbindungen 1.1, 1.2, 1.4, 1.5, 1.7, 1.8, 1.19 und 1.41 zeigen eine Wirkung über 80 %.

## Patentansprüche

1. Eine Verbindung der Formel

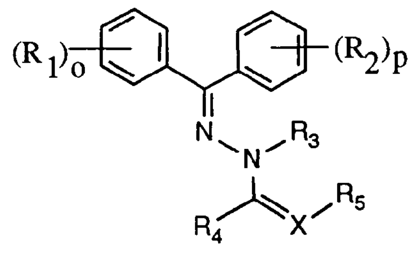

(I),

worin
o und p unabhängig voneinander 0, 1, 2, 3, 4 oder 5, wobei, wenn o grösser als 1 ist, die Reste $R_1$ gleich oder verschieden sind und wobei, wenn p grösser als 1 ist, die Reste $R_2$ gleich oder verschieden sind;
$R_1$ und $R_2$ unabhängig voneinander $C_1$-$C_4$-Alkyl, Halogen-$C_1$-$C_4$-alkyl, Halogen, -$NO_2$, -OH, $C_1$-$C_4$-Alkoxy, Halogen-$C_1$-$C_4$-alkoxy, $C_1$-$C_4$-Alkylthio, Halogen-$C_1$-$C_4$-alkylthio, -O-S(=O)-$R_6$ ,-O-S(=O)$_2$-$R_6$, Phenoxy oder -N($R_{11}$)$SO_2R_{12}$ und/oder zwei an benachbarte C-Atome des Phenylrings gebundene Substituenten $R_1$ und/oder zwei an benachbarte C-Atome des Phenylrings gebundene Substituenten $R_2$ unabhängig voneinander gemeinsam -Y-Z-Y-;
$R_3$ Wasserstoff, $C_1$-$C_4$-Alkyl oder Halogen-$C_1$-$C_4$-alkyl;
$R_4$ Wasserstoff, $C_1$-$C_4$-Alkyl, Halogen-$C_1$-$C_4$-alkyl, unsubstituiertes Phenyl oder Naphthyl oder ein- oder zweifach substituiertes Phenyl oder Naphthyl, wobei die Substituenten ausgewählt sind aus der Gruppe, bestehend aus Halogen, $C_1$-$C_4$-Alkyl, Halogen-$C_1$-$C_4$-alkyl, $C_1$-$C_4$-Alkoxy, Halogen-$C_1$-$C_4$-alkoxy, $C_1$-$C_4$-Alkylthio, Halogen-$C_1$-$C_4$-alkylthio, -$NO_2$ und -CN;
$R_5$ -S-$R_7$, -S(=O)-$R_7$, -S(=O)$_2$-$R_7$, -$NO_2$, -CN, -C(=O)-$R_8$ oder -C(=O)-O$R_6$;
$R_6$ $C_1$-$C_8$-Alkyl, oder Halogen-$C_1$-$C_8$-alkyl oder Phenyl;
$R_7$ $C_1$-$C_8$-Alkyl, $C_3$-$C_6$-Cycloalkyl, Halogen-$C_1$-$C_8$-alkyl, unsubstituiertes oder ein- oder zweifach substituiertes Phenyl, wobei die Substituenten ausgewählt sind aus der Gruppe, bestehend aus Halogen, $C_1$-$C_4$-Alkyl, Halogen-$C_1$-$C_4$-alkyl, $C_1$-$C_4$-Alkoxy, Halogen-$C_1$-$C_4$-alkoxy, $C_1$-$C_4$-Alkylthio, Halogen-$C_1$-$C_4$-alkylthio, -$NO_2$ und -CN; Benzyl oder unsubstituiertes oder ein- oder zweifach substituiertes Amino, wobei die Substituenten ausgewählt sind aus der Gruppe, bestehend aus $C_1$-$C_4$-Alkyl, Halogen-$C_1$-$C_4$-alkyl oder Phenyl;
$R_8$ $C_1$-$C_8$-Alkyl, Halogen-$C_1$-$C_8$-alkyl oder unsubstituiertes oder ein- oder zweifach substituiertes Phenyl, wobei die Substituenten ausgewählt sind aus der Gruppe, bestehend aus Halogen, $C_1$-$C_4$-Alkyl, Halogen-$C_1$-$C_4$-alkyl, $C_1$-$C_4$-Alkoxy, Halogen-$C_1$-$C_4$-alkoxy, $C_1$-$C_4$-Alkylthio, Halogen-$C_1$-$C_4$-alkylthio, -$NO_2$ und -CN;
X N oder C($R_9$);
Y unabhängig voneinander O oder S;
Z Methylen, Eth-1,2-ylen, Halogenmethylen oder Halogeneth-1,2-ylen;
$R_9$ Wasserstoff, $C_1$-$C_4$-Alkyl, Halogen-$C_1$-$C_4$-alkyl, -CN, -C(=O)-$R_{10}$ oder -C(=O)-O$R_{10}$;
$R_{10}$ Wasserstoff, $C_1$-$C_8$-Alkyl oder Halogen-$C_1$-$C_8$-alkyl;
$R_{11}$ Wasserstoff, $C_1$-$C_4$-Alkyl oder Halogen-$C_1$-$C_4$-alkyl; und
$R_{12}$ $C_1$-$C_4$-Alkyl, Halogen-$C_1$-$C_4$-alkyl, unsubstituiertes Phenyl oder ein- oder zweifach substituiertes Phenyl bedeuten, wobei die Substituenten ausgewählt sind aus der Gruppe bestehend aus Halogen, $C_1$-$C_4$-Alkyl, Halogen-$C_1$-$C_4$-alkyl, $C_1$-$C_4$-Alkoxy, Halogen-$C_1$-$C_4$-alkoxy, $C_1$-$C_4$-Alkylthio, Halogen-$C_1$-$C_4$-alkylthio, -$NO_2$ und -CN;
und gegebenenfalls Tautomere davon, sowie deren Salze und die Salze der Tautomeren; mit der Massgabe (A), dass in Verbindungen der Formel I in freier Form, worin o und p jeweils für 0 stehen, $R_4$ Wasserstoff ist und X für N steht, $R_3$ von Wasserstoff verschieden ist, wenn $R_6$ Methansulfonyl, unsubstituiertes Phenylsulfonyl oder 4-Methylphenylsulfonyl darstellt und mit der weiteren Massgabe
(B), dass in Verbindungen der Formel I in freier Form, worin o und p jeweils für 1 stehen, $R_1$ Methansulfonyloxy ist, $R_2$ Chlor ist, $R_4$ Methyl ist, X für C($R_9$) steht und $R_9$ Wasserstoff ist, $R_3$ von Wasserstoff verschieden ist, wenn $R_5$ Ethoxycarbonyl, Methoxycarbonyl oder Cyano darstellt.

2. Eine Verbindung gemäss Anspruch 1 der Formel I in freier Form.

3. Eine Verbindung gemäss Anspruch 1 der Formel I, worin o 1 oder 2 ist, wobei, wenn o 2 ist, die Reste $R_1$ gleich oder verschieden sind, und
$R_1$ Halogen, $-NO_2$, -OH, $-O-S(=O)_2-C_1-C_4$-Alkyl, $-O-S(=O)_2$-Halogen-$C_1$-$C_4$-alkyl, Phenoxy, $-NO_2$ oder $-N(R_{11})SO_2R_{12}$, oder zwei an benachbarte C-Atome des Phenylrings gebundene Substituenten $R_1$ gemeinsam -O-Methylen-O- oder -O-Halogenmethylen-O-, $R_{11}$ Wasserstoff oder $C_1$-$C_4$-Alkyl, und $R_{12}$ Halogen-$C_1$-$C_4$-alkyl bedeutet, oder gegebenenfalls ein Tautomeres davon.

4. Eine Verbindung gemäss Anspruch 3 der Formel I, worin
$R_1$ Halogen, -OH, $-O-S(=O)_2-C_1-C_4$-Alkyl, $-O-S(=O)_2$-Halogen-$C_1$-$C_4$-alkyl, Phenoxy, $-N(R_{11})SO_2$-halogen-$C_1$-$C_2$-alkyl oder zwei an benachbarte C-Atome des Phenylrings gebundene Substituenten $R_1$ gemeinsam -O-Methylen-O- oder -O-Halogenmethylen-O- und $R_{11}$ Wasserstoff oder $C_1$-$C_2$-alkyl bedeutet, oder gegebenenfalls ein Tautomeres davon.

5. Eine Verbindung gemäss Anspruch 1, 3 oder 4 der Formel I, worin
p 0, 1 oder 2, wobei, wenn p 2 ist, die Reste $R_2$ gleich oder verschieden sind, und $R_2$ Halogen, -OH, $-O-S(=O)_2-C_1-C_4$-Alkyl oder $-O-S(=O)_2$-Halogen-$C_1$-$C_4$-alkyl oder zwei an benachbarte C-Atome des Phenylrings gebundene Substituenten $R_2$ gemeinsam -O-Methylen-O- oder -O-Halogenmethylen-O-, oder gegebenenfalls ein Tautomeres davon.

6. Eine Verbindung gemäss Anspruch 5 der Formel I, worin
p 0, 1 oder 2, wobei, wenn p 2 ist, die Reste $R_2$ gleich sind, und $R_2$ Halogen, -OH, $-O-S=(=O)_2-C_1-C_2$-Alkyl oder $-O-S(=O)_2$-Halogen-$C_1$-$C_2$-alkyl oder zwei an benachbarte C-Atome des Phenylrings gebundene Substituenten $R_2$ gemeinsam -O-Halogenmethylen-O- sind, oder gegebenenfalls ein Tautomeres davon.

7. Eine Verbindung gemäss Anspruch 1 oder 3 bis 6 der Formel I, worin
$R_3$ Wasserstoff oder $C_1$-$C_4$-Alkyl bedeutet, oder gegebenenfalls ein Tautomeres davon.

8. Eine Verbindung gemäss Anspruch 1 oder 3 bis 7 der Formel I, worin
$R_4$ Wasserstoff, $C_1$-$C_4$-Alkyl, Halogen-$C_1$-$C_4$-alkyl oder Phenyl bedeutet, oder gegebenenfalls ein Tautomeres davon.

9. Eine Verbindung gemäss Anspruch 1 oder 3 bis 8 der Formel I, worin
$R_5$ $-S(=O)_2-R_7$, -CN, $-C(=O)-R_8$ oder $-C(=O)-OR_8$,
$R_7$ $C_1$-$C_6$-Alkyl, $C_3$-$C_6$-Cycloalkyl, Halogen-$C_1$-$C_4$-alkyl, Benzyl, $C_1$-$C_4$-Dialkylamino oder unsubstituiertes oder einfach durch Halogen, $C_1$-$C_4$-Alkyl oder $C_1$-$C_4$-Alkoxy substituiertes Phenyl,
$R_8$ $C_1$-$C_4$-Alkyl, Halogen-$C_1$-$C_4$-alkyl oder Phenyl,
X N oder $C(R_9)$,
$R_9$ Wasserstoff, -CN oder $-C(=O)-OR_{10}$ und
$R_{10}$ $C_1$-$C_4$-Alkyl bedeutet, oder gegebenenfalls ein Tautomeres davon.

10. Eine Verbindung gemäss Anspruch 9 der Formel I, worin
$R_5$ $-S(=O)_2-R_7$, -CN, $-C(=O)-R_8$ oder $-C(=O)-OR_8$,
$R_7$ $C_1$-$C_4$-Alkyl, Halogen-$C_1$-$C_2$-alkyl, Benzyl oder Dimethylamino, oder unsubstituiertes oder einfach durch Halogen, $C_1$-$C_2$-Alkyl oder $C_1$-$C_2$-Alkoxy substituiertes Phenyl,
$R_8$ $C_1$-$C_4$-Alkyl oder Halogen-$C_1$-$C_2$-alkyl,
X N oder $C(R_9)$,
$R_9$ Wasserstoff, -CN oder $-C(=O)-OR_{10}$ und
$R_{10}$ $C_1$-$C_4$-Alkyl bedeutet, oder gegebenenfalls ein Tautomeres davon.

11. Eine Verbindung gemäss Anspruch 1 der Formel I, worin
o 1 oder 2,
p 0, 1 oder 2, wobei, wenn o 2 ist, die Reste $R_1$ gleich oder verschieden sind und wobei, wenn p 2 ist, die Reste $R_2$ gleich oder verschieden sind;
$R_1$ und $R_2$ unabhängig voneinander Halogen, $-NO_2$, -OH, Phenoxy, $-N(R_{11})SO_2CF_3$ oder $-O-S(=O)_2-R_8$ oder zwei an benachbarte C-Atome des Phenylrings gebundene Substituenten $R_1$ oder zwei an benachbarte C-Atome des Phenylrings gebundene Substituenten $R_2$ gemeinsam -O-Z-O-;
$R_3$ Wasserstoff oder $C_1$-$C_4$-Alkyl;
$R_4$ Wasserstoff, $C_1$-$C_4$-Alkyl, Halogen-$C_1$-$C_4$-alkyl oder unsubstituiertes Phenyl;
$R_5$ $-S(=O)_2-R_7$, -CN, $-C(=O)-R_8$ oder $-C(=O)-OR_8$;

$R_6$ $C_1$-$C_4$-Alkyl, Halogen-$C_1$-$C_4$-alkyl oder Phenyl;
$R_7$ $C_1$-$C_6$-Alkyl, $C_3$-$C_6$-Cycloalkyl, Halogen-$C_1$-$C_4$-alkyl, unsubstituiertes oder einfach durch Halogen, $C_1$-$C_4$-Alkyl oder $C_1$-$C_4$-Alkoxy substituiertes Phenyl, Benzyl oder Dimethylamino;
$R_8$ $C_1$-$C_4$-Alkyl oder Halogen-$C_1$-$C_4$-alkyl;
X N oder C($R_9$);
Z Methylen oder Halogenmethylen;
$R_9$ Wasserstoff, -CN, -C(=O)-$R_{10}$ oder -C(=O)-O$R_{10}$;
$R_{10}$ $C_1$-$C_4$-Alkyl und
$R_{11}$ Wasserstoff, Methyl oder Ethyl bedeuten, oder gegebenenfalls ein Tautomeres davon.

**12.** Eine Verbindung gemäss Anspruch 11 der Formel I, worin
o 1 oder 2,
p 0, 1 oder 2, wobei, wenn o 2 ist, die Reste $R_1$ gleich oder verschieden sind und wobei, wenn p 2 ist, die Reste $R_2$ gleich oder verschieden sind;
$R_1$ und $R_2$ unabhängig voneinander Halogen, -OH oder -O-S(=O)$_2$-$R_6$ oder zwei an benachbarte C-Atome des Phenylrings gebundene Substituenten $R_1$ oder zwei an benachbarte C-Atome des Phenylrings gebundene Substituenten $R_2$ gemeinsam -O-Z-O-;
$R_3$ Wasserstoff oder $C_1$-$C_4$-Alkyl;
$R_4$ Wasserstoff, $C_1$-$C_4$-Alkyl, Halogen-$C_1$-$C_4$-alkyl oder unsubstituiertes Phenyl;
$R_5$ -S(=O)$_2$-$R_7$, -CN, -C(=O)-$R_8$ oder -C(=O)-O$R_6$;
$R_6$ $C_1$-$C_4$-Alkyl oder Halogen-$C_1$-$C_4$-alkyl;
$R_7$ $C_1$-$C_6$-Alkyl, $C_3$-$C_6$-Cycloalkyl, Halogen-$C_1$-$C_4$-alkyl oder unsubstituiertes oder einfach durch Halogen, $C_1$-$C_4$-Alkyl oder $C_1$-$C_4$-Alkoxy substituiertes Phenyl, Benzyl oder Dimethylamino;;
$R_8$ $C_1$-$C_4$-Alkyl oder Halogen-$C_1$-$C_4$-alkyl;
X N oder C($R_9$);
Z Methylen oder Halogenmethylen;
$R_9$ Wasserstoff, -CN, -C(=O)-$R_{10}$ oder -C(=O)-O$R_{10}$; und
$R_{10}$ $C_1$-$C_4$-Alkyl bedeuten, oder gegebenenfalls ein Tautomeres davon.

**13.** Eine Verbindung gemäss Anspruch 1 der Formel I, ausgewählt aus der Gruppe, bestehend aus den Verbindungen

(a) 1-(4-Chlorphenyl)-5-ethansulfonyl-2,3,5-triaza-1-(4-trifluormethansulfonyloxyphenyl)-penta-1,4-dien bzw. 1-(4-Chlorphenyl)-5-ethansulfonyl-2,3,5-triaza1-(4-trifluormethansulfonyloxyphenyl)-penta-1,3-dien; und

(b) 1-(4-Chlorphenyl)-2,3-diaza-4-methyl-6-oxo-1-(4-trifluormethansulfonyloxyphenyl)-hepta-1,4-dien bzw. 1-(4-Chlorphenyl)-2,3-diaza-4-methyl-6-oxo-1-(4-trifluormethansulfonyloxyphenyl)-hepta-1,3-dien.

**14.** Verfahren zur Herstellung einer Verbindung gemäss Anspruch 1 der Formel I oder gegebenenfalls eines Tautomeren davon, jeweils in freier Form oder in Salzform, dadurch gekennzeichnet, dass man
a) eine Verbindung der Formel

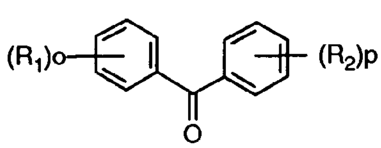

(II),

worin o, p, $R_1$ und $R_2$ die für die Formel I angegebenen Bedeutungen haben, vorzugsweise in Gegenwart einer Säure, mit einer Verbindung der Formel

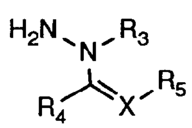

(III),

worin $R_3$, $R_4$, $R_6$ und X die für die Formel I angegebenen Bedeutungen haben, oder einem Salz und/oder

gegebenenfalls einem Tautomeren davon umsetzt oder
b) eine Verbindung der Formel

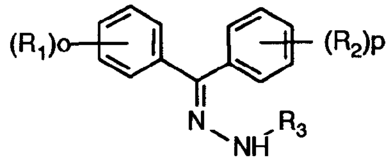

(IV),

worin o, p, $R_1$, $R_2$ und $R_3$ die für die Formel I angegebenen Bedeutungen haben, oder ein Salz davon, vorzugsweise in Gegenwart einer Säure oder einer Base, mit einer Verbindung der Formel

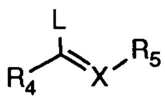

(V),

worin L Hydroxy, $C_1$-$C_8$-Alkoxy, Halogen-$C_1$-$C_8$-alkoxy, $C_1$-$C_8$-Alkanoyloxy, Mercapto, $C_1$-$C_8$-Alkylthio, Halogen-$C_1$-$C_8$-alkylthio, $C_1$-$C_8$-Alkansulfonyloxy, Halogen-$C_1$-$C_8$-alkansulfonyloxy, Benzolsulfonyloxy, Toluolsulfonyloxy oder Halogen ist und $R_4$, $R_5$ und X die für die Formel I angegebenen Bedeutungen haben, oder einem Salz und/oder gegebenenfalls einem Tautomeren davon umsetzt
und jeweils, wenn erwünscht, eine verfahrensgemäss oder auf andere Weise erhältliche Verbindung der Formel I oder ein Tautomeres davon, jeweils in freier Form oder in Salzform, in eine andere Verbindung der Formel I oder ein Tautomeres davon überführt, ein verfahrensgemäss erhältliches Gemisch von Isomeren auftrennt und das gewünschte Isomere isoliert und/oder eine verfahrensgemäss erhältliche freie Verbindung der Formel I oder ein Tautomeres davon in ein Salz oder ein verfahrensgemäss erhältliches Salz einer Verbindung der Formel I oder eines Tautomeren davon in die freie Verbindung der Formel I oder ein Tautomeres davon oder in ein anderes Salz überführt.

**15.** Schädlingsbekämpfungsmittel, dadurch gekennzeichnet, dass es mindestens eine Verbindung gemäss einem der Ansprüche 1 bis 13 der Formel I oder gegebenenfalls ein Tautomeres davon, jeweils in freier Form oder in agrochemisch verwendbarer Salzform, als Wirkstoff und mindestens einen Hilfsstoff enthält, wobei die in Anspruch 1 erwähnte Massgabe (A) nicht gültig ist.

**16.** Mittel gemäss Anspruch 15 zur Bekämpfung von Insekten.

**17.** Verfahren zur Herstellung eines Mittels gemäss Anspruch 15, dadurch gekennzeichnet, dass man den Wirkstoff mit dem (den) Hilfsstoff(en) innig vermischt.

**18.** Verfahren zur Bekämpfung von Schädlingen, dadurch gekennzeichnet, dass man als Wirkstoff eine Verbindung gemäss einem der Ansprüche 1 bis 13 der Formel I oder gegebenenfalls ein Tautomeres davon, jeweils in freier Form oder in agrochemisch verwendbarer Salzform, wobei die in Anspruch 1 erwähnte Massgabe (A) nicht gültig ist, oder ein Mittel gemäss Anspruch 15 auf die Schädlinge oder ihren Lebensraum appliziert.

**19.** Verfahren gemäss Anspruch 18 zur Bekämpfung von Insekten.

Europäisches Patentamt

# EUROPÄISCHER RECHERCHENBERICHT

Nummer der Anmeldung

EP 93 81 0461 9

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| A | EP-A-0 026 040 (THE BOOTS CO LTD)<br>* Tabelle I, Verbindungen 12, 13, 43; Ansprüche 1-6, 8-14 *<br>--- | 1,14,19 | C07C311/51<br>C07C309/65<br>C07D317/46<br>C07C251/86<br>A01N41/04<br>A01N41/06<br>A01N43/30<br>A01N35/10 |
| A | EP-A-0 003 913 (THE BOOTS COMPANY LTD)<br>* Ansprüche 1-17,20 *<br>--- | 1,14-19 | |
| A | EP-A-0 355 832 (SUMITOMO CHEMICAL CO LTD)<br>* Ansprüche 1,3,6-8 *<br>--- | 1,14-19 | |
| A | ACTA CHEM. SCAND.<br>Bd. B34, Nr. 3, 1980,<br>Seiten 233 - 234<br>P. JAKOBSEN ET AL<br>* Seite 233, Tabelle I, Verbindungen 1e, 1l, 1s; Spalte 2, eperimenteller Teil *<br>----- | 1,14 | |
| | | | **RECHERCHIERTE SACHGEBIETE (Int. Cl.5 )** |
| | | | C07C<br>C07D |

Der vorliegende Recherchenbericht wurde für alle Patentansprüche erstellt

| Recherchenort | Abschlußdatum der Recherche | Prüfer |
|---|---|---|
| BERLIN | 15 NOVEMBER 1993 | VAN AMSTERDAM L. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)